(19) Europäisches Patentamt
European Patent Office
Office européen des brevets

(11) Veröffentlichungsnummer: **0 271 865 B1**

# (12) EUROPÄISCHE PATENTSCHRIFT

(45) Veröffentlichungstag der Patentschrift: **07.10.92**

(51) Int. Cl.⁵: **C07K 5/08**, C07D 261/02, A61K 37/64

(21) Anmeldenummer: **87118530.2**

(22) Anmeldetag: **15.12.87**

## (54) 2,3-Disubstituierte Isoxazolidine, Verfahren zu ihrer Herstellung, diese enthaltende Mittel und ihre Verwendung.

(30) Priorität: **17.12.86 DE 3643012**

(43) Veröffentlichungstag der Anmeldung:
**22.06.88 Patentblatt 88/25**

(45) Bekanntmachung des Hinweises auf die Patenterteilung:
**07.10.92 Patentblatt 92/41**

(84) Benannte Vertragsstaaten:
**AT BE CH DE ES FR GB GR IT LI LU NL SE**

(56) Entgegenhaltungen:

**CHEMICAL ABSTRACTS, Band 99, Nr. 19, 7. November 1983, Seite 654, Zusammenfassung Nr. 158804u, Columbus, Ohio, US; A. VASELLA et al.: "Synthesis of D- and L-5-oxaproline and a new captepril analog", & HELV. CHIM. ACTA 1983, 66(4), 1241-52**

(73) Patentinhaber: **HOECHST AKTIENGESELLSCHAFT**
**Postfach 80 03 20**
**W-6230 Frankfurt am Main 80(DE)**

(72) Erfinder: **Henke, Stephan, Dr.**
**Sophienruhe 4**
**W-6232 Bad Soden am Taunus 2(DE)**
Erfinder: **Brocks, Dietrich, Dr.**
**Goethering 9**
**W-6200 Wiesbaden 42(DE)**
Erfinder: **Günzler, Volkmar, Dr.**
**Marburger Strasse 2**
**W-3550 Marburg-Cappel(DE)**

EP 0 271 865 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 95, Nr. 7, 17. August 1981, Seite 706, Zusammenfassung Nr. 62045p, Columbus, Ohio, US; A. VASELLA et al.: "Asymmetric synthesis of a new proline analog", & J. CHEM. SOC., CHEM. COMMUN. 1981, (3), 97-8

THE JOURNAL OF BIOLOGICAL CHEMISTRY, Band 263, Nr. 36, 25. Dezember 1988, Seiten 19498-19504, The American Society for Biochemistry and Molecular Biology, Inc., Bethesda, Maryland, US; V. GÜNZLER et al.: "Syncatalytic inactivation of prolyl 4-hydroxylase by synthetic peptides containing the unphysiologic amino acid-5-oxaproline"

**Beschreibung**

Aus dem US-Patent 4 457 936 sind Hydroxyphenylthiazol-, -thiazolin- und -thiazolidin-carbonsäuren und ihre Verwendung als Inhibitoren u.a. der Prolylhydroxylase bekannt.

Es wurde gefunden, daß in bestimmter Weise substituierte Isoxazolidinderivate hochwirksame Suizidinhibitoren der Prolylhydroxylase sind.

Die Erfindung betrifft daher Verbindungen der Formel I

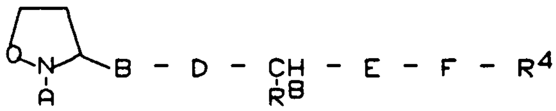

(I)

in welcher

A

a$_1$) ($C_1$-$C_8$)-Alkyl,
($C_1$-$C_8$)-Alkanoyl,
($C_1$-$C_8$)-Alkoxycarbonyl oder
($C_1$-$C_8$)-Alkylsulfonyl bedeutet,
in welchen jeweils 1, 2 oder 3 Wasserstoffatome gegebenenfalls durch 1, 2 oder 3 gleiche oder verschiedene Reste aus der Reihe
Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$)-Aryl-($C_1$-$C_5$)-alkyl ersetzt sind, oder in welchen jeweils 1 Wasserstoffatom gegebenenfalls durch einen Rest aus der Reihe
($C_3$-$C_8$)-Cycloalkyl,
($C_1$-$C_4$)-Alkylsulfonyl,
($C_1$-$C_4$)-Alkylsulfinyl,
($C_6$-$C_{12}$)-Aryl-($C_1$-$C_4$)-alkylsulfonyl,
($C_6$-$C_{12}$)-Aryl-($C_1$-$C_4$)-alkylsulfinyl,
($C_6$-$C_{12}$)-Aryloxy,
($C_3$-$C_9$)-Heteroaryl und
($C_3$-$C_9$)-Heteroaryloxy
und
1 oder 2 Wasserstoffatome durch 1 oder 2 gleiche oder verschienden Reste aus der Reihe Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$)-Aryl-($C_1$-$C_5$)-alkyl
ersetzt sind,
a$_2$) ($C_3$-$C_8$)-Cyloalkyl,

($C_6$-$C_{12}$)-Aryl,
($C_6$-$C_{12}$)-Arylsulfonyl oder
($C_3$-$C_9$)-Heteroaryl bedeutet,
wobei in den unter $a_1$) und $a_2$) definierten Resten jeweils ($C_6$-$C_{12}$)-Aryl bzw. ($C_3$-$C_9$)-Heteroaryl gegebenenfalls durch 1, 2 oder 3 gleiche oder veschiedene Reste aus der Reihe
Carboxy,
Amino,
Nitro,
($C_1$-$C_4$)-Alkylamino,
Hydroxy,
($C_1$-$C_4$)-Alkoxy,
Halogen,
Cyano,
Di-($C_1$-$C_4$)-alkylamino,
Carbamoyl,
Sulfamoyl,
($C_1$-$C_4$)-Alkoxycarbonyl,
substituiert ist, oder
$a_3$) eine Rest der Formel IIa oder IIb bedeutet,

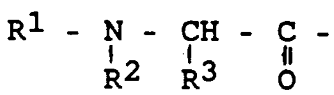

(IIa)

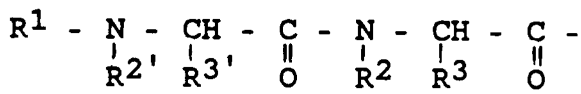

(IIb)

$R^1$
$b_1$) Wasserstoff bedeutet oder
$b_2$) wie A unter $a_1$) oder $a_2$) definiert ist,
$c_1$) $R^2$ und $R^{2'}$ gleich oder verschieden sind und Wasserstoff oder Methyl bedeuten,
$R^3$ und $R^{3'}$ gleich oder verschieden sind und Wasserstoff oder
($C_1$-$C_6$)-Alkyl, vorzugsweise ($C_1$-$C_4$)-Alkyl, das gegebenenfalls durch
Amino,
Benzyloxycarbonylamino,
Hydroxy,
Carboxy,
Carbamoyl,
Guanidino,
Ureido,
Mercapto,
Methylmercapto,
Phenyl,
4-Chlorphenyl,
4-Fluorphenyl,
4-Nitrophenyl,
4-Methoxyphenyl,
4-Hydroxyphenyl,
Phthalimido,
4-Imidazolyl,
3-Indolyl,
2-Thienyl,
3-Thienyl,
2-Pyridyl,
3-Pyridyl oder
Cyclohexyl monosubstituiert ist, bedeuten,
$c_2$) $R^2$ und $R^3$ und/oder $R^{2'}$ und $R^{3'}$ jeweils zusammen für eine [-$CH_2$-$CH_2$-$CH_2$-]-Kette stehen, worin

eine $CH_2$-Gruppe durch O ersetzt eine kann, oder
$c_3$) $R^2$ und $R^3$ und/oder $R^{2'}$ und $R^{3'}$ jeweils zusammen für

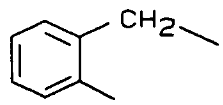

stehen,
B Carbonyl,
Thiocarbonyl,
Carbimidoyl
N-($C_1$-$C_3$)-Alkylcarbimidoyl,
N-($C_1$-$C_3$)-Alkoxycarbimidoyl,
Sulfinyl,
$CR^5R^6$ oder
eine direkte Bindung bedeutet,
$d_1$) $R^5$ und $R^6$ gleich oder verschieden sind und Wasserstoff,
($C_1$-$C_8$)-Alkyl oder
($C_3$-$C_8$)-Cycloalkyl bedeuten oder
$d_2$) $R^5$ und $R^6$ gemeinsam für $-[CH_2]_m-$ mit m = 4 oder 5 stehen, worin 1 oder 2 $CH_2$-Gruppen gegebenenfalls durch O, S und/oder $NR^7$ ersetzt sind,
D Imino,
N-Methylimino,
Oxy,
Methylen oder
eine direkte Bindung bedeutet,
E Carbonyl,
$C=NR^7$,
$C=N-OR^7$ oder
Sulfinyl bedeutet oder, falls F für eine Bindung steht, acuh Hydroxymethylen bedeuten kann,
$R^7$ wie $R^5$ unter $d_1$) definiert ist,
F Oxy,
Imino,
N-Methylimino oder
eine direkte Bindung bedeutet,
$R^4$ ($C_1$-$C_6$)-Alkyl,
($C_3$-$C_6$)-Cycloalkyl,
($C_6$-$C_{12}$)-Aryl,
($C_6$-$C_{12}$)-Aryl-($C_1$-$C_5$)-alkyl,
($C_6$-$C_{12}$)-Aryloxy-($C_1$-$C_5$)-alkyl,
($C_3$-$C_9$)-Heterorayl oder
($C_3$-$C_9$)-Heterorayl-($C_1$-$C_5$)-alkyl bedeutet, worin jeweils Alkyl gegebenenfalls durch 1 oder 2 gleiche oder verschiedene Reste aus der Reihe
Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$)-Aryl-($C_1$-$C_5$)-alkyl substituiert ist und worin jeweils ($C_6$-$C_{12}$)-Aryl bzw.
($C_3$-$C_9$)-Heteroaryl gegebenenfalls durch 1, 2 oder 3 gleiche oder verschiedene Reste aus der Reihe

Carboxy,
Cyano,
Amino,
Nitro,
$(C_1-C_4)$-Alkylamino,
Hydroxy,
$(C_1-C_4)$-Alkoxy,
Halogen,
Di-$(C_1-C_4)$-alkylamino,
Carbamoyl,
Sulfamoyl und
$(C_1-C_4)$-Alkoxycarbonyl substituiert ist,
und
$R^8$ wie $R^3$ unter $c_1$) definiert ist,

sowie deren physiologisch verträgliche Salze.

Unter $(C_6-C_{12})$-Aryl wird beispielsweise Phenyl, Naphthyl oder Biphenylyl verstanden. Entsprechendes gilt für davon abgeleitete Reste wie Aroyl.

Alkyl und davon abgeleitete Reste wie Alkoxy können geradkettig oder verzweigt sein.

Halogen steht vorzugsweise für Fluor, Chlor oder Brom.

Ein Heteroaryl-Rest im Sinne der vorliegenden Erfindung ist der Rest eines monocyclischen oder bicyclischen $(C_3-C_9)$-Heteroaromaten, der im Ringsystem ein oder zwei N-Atome und/oder ein S- oder ein O-Atom erhält. Zum Begriff "Heteroaromat" siehe Garatt, Vollhardt, Aromatizität, Stuttgart 1973, Seiten 131 - 153. Beispiele geeigneter Heteroarylreste sind die Reste von Thiophen, Furan, Benzo-[b]thiophen, Benzofuran, Pyrrol, Imidazol, Pyrazol, Pyridin, Pyrazin, Pyrimidin, Pyridazin, Indol, Chinolin, Isochinolin, Oxazol, Isoxazol, Thiazol, Isothiazol, Isobenzofuran, Indolizin, Isoindol, Indazol, Phthalazin, Naphthyridin, Chinoxalin, Chinazolin, Cinnolin und Furazan. Aryl, Alkyl, Heteroaryl und davon abgeleitete Reste können wie oben angegeben einfach oder, falls chemisch möglich, auch mehrfach substituiert sein.

Chiralitätszentren können, wenn nicht anders angegeben, in der R- oder in der S-Konfiguration vorliegen. Die Erfindung betrifft sowohl die optisch reinen Verbindungen als auch Stereoisomerengemische wie Enantiomerengemische und Diastereomerengemische.

Als Salze kommen insbesondere Alkali- und Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Bevorzugt sind solche Verbindungen der Formel I, worin A gegebenenfalls substituiertes $(C_1-C_8)$-Alkyl, $(C_1-C_8)$-Alkanoyl oder gegebenenfalls substituiertes $(C_1-C_8)$-Alkoxycarbonyl bedeutet oder definiert ist, wie oben unter $a_3$), solche, worin $R^8$ Wasserstoff bedeutet und solche, worin B Carbonyl, Thiocarbonyl oder $CR^5R^6$ mit $R^5$ und $R^6$ = H bedeutet.

Weiterhin sind Verbindungen der Formel I bevorzugt,
in der D Imino, Oxy oder Methylen - besonders bevorzugt Imino - bedeutet,
E Carbonyl bedeutet,
F Oxy oder eine direkte Bindung bedeutet und/oder
$R^4$ gegebenenfalls substituiertes $(C_6-C_{12})$-Aryl oder gegebenenfalls im Alkylteil und/oder gegebenenfalls im Arylteil substituiertes $(C_6-C_{12})$-Aryl-$(C_1-C_5)$-alkyl bedeutet, wobei als Substituenten des Arylteils insbesondere Halogene, Pseudohalogene und Carboxy-Gruppen, als Substituenten des Alkylteiles insbesondere Carboxy-, $(C_1-C_4)$-Alkoxycarbonyl-und Aminogruppen in Frage kommen.

Besonders bevorzugt sind Verbindungen der Formel I, worin A für substituiertes $(C_1-C_8)$-Alkanoyl, wobei als Substituenten beispielsweise $(C_6-C_{12})$-Aryl-$(C_1-C_4)$-alkylsulfonyl und $(C_6-C_{12})$-Aryl-$(C_1-C_4)$-alkylsulfinyl in Frage kommen, oder für einen Rest der Formel IIa, wie oben unter $a_3$) definiert, steht.

Die Erfindung betrifft auch ein Verfahren zur Herstellung von Verbindungen der Formel I, das dadurch gekennzeichnet ist, daß man die Verbindungen in bekannter Weise aus den Fragmenten beispielsweise durch deren Kupplung aufbaut, gegebenenfalls eine oder mehrere temporär eingeführte Schutzgruppe(n) abspaltet, Carbonylfunktionen gegebenenfalls in die Thia-Analogen überführt und die so erhaltenen Verbindungen der Formel I gegebenenfalls in ihre physiologisch verträglichen Salze überführt.

Unter Fragmenten im obigen Sinne werden Aminosäuren, mehrere Aminosäuren enthaltende Segemente, Derivate von Aminosäuren, Derivate von Peptiden mit modifizierten Peptidbindungen ebenso wie verschiedenartig substituierte Carbonsäuren, diverse Alkohole und deren Derivate verstanden.

Die Kupplung kann dadurch erfolgen, daß man z.B. ein Fragment einer Verbindung der Formel I mit einer endständigen Carboxylgruppe oder einem reaktiven Säurederivat mit einem entsprechenden anderen

Fragment, welches z.B. eine freie Aminogruppe enthält, wobei eventuell vorhandene nicht an der Reaktion beteiligte funktionelle Gruppen gegebenenfalls geschützt sein können, unter Bildung einer Amidbindung in einem inerten Lösungsmittel kondensiert. Methoden, die zur Bildung einer Amidbindung geeignet sind werden in Houben-Weyl, Methoden der organischen Chemie, Band 15/2 beschrieben.

Man führt das Verfahren vorteilhaft in der Weise durch, daß man

a) eine Verbindung der Formel IIIa mit einer Verbindung der Formel IIIb

$A - X$

(IIIa)

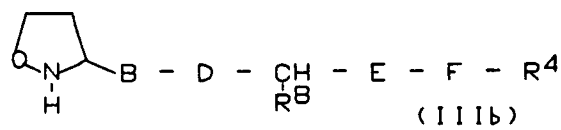

(IIIb)

in welcher A, B, D, E, F, $R^4$ und $R^8$ wie oben definiert sind und X für eine nucleophil ablösbare Abgangsgruppe wie OH, Cl, Br, I, Tosyl, Triflat oder, falls A ein Acylrest ist, auch für eine Aktivestergruppe steht, kondensiert,

b) eine Verbindung der Formel IVa mit einer Verbindung der Formel IVb,

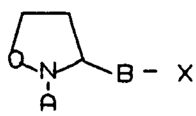

(IVa)

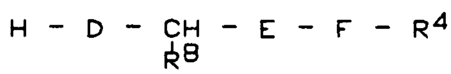

(IVb)

in welcher A, B, E, F, $R^4$ und $R^8$ wie oben definiert sind, D für Imino, N-Methylimino oder Oxy steht und X für eine nucleophil ablösbare Abgangsgruppe wie OH, Cl, BR, I, Tosyl, Triflat oder, falls B Carbonyl bedeutet, auch für eine Aktivestergruppe steht, kondensiert oder

c) eine Verbindung der Formel Va mit einer Verbindung der Formel Vb,

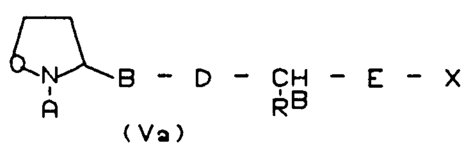

(Va)

$H - F - R^4$

(Vb)

in welchen A, B, D, $R^4$ und $R^8$ wie oben definiert sind, E mit Ausnahme von Hydroxymethylen wie oben definiert ist, F Oxy, Imino oder N-Methylimino bedeutet und X für eine nucleophil ablösbare Abgangsgruppe wie OH, Cl, Br, I, Tosyl, Triflat oder eine Aktivestergruppe steht, kondensiert.

Die Umsetzung einer Carbonsäure der Formel IIIa, IVa, bzw. Va mit der entsprechenden Verbindung der Formel IIIb, IVb bzw. Vb mit freier Aminogruppe wird vorzugsweise in einem in der Peptidchemie üblichen Lösungsmittel oder auch in Wasser/Lösungsmittel-Gemischen in Gegenwart eines geeigneten Kondensationsmittels durchgeführt, wie z.B.

1. Dicyclohexylcarbodiimid unter Zusatz von 1-Hydroxybenzotriazol (DCC/HOBt-Methode, Lit.: Chem. Ber. 103 (1970) 788)

2. Dicyclohexylcarbodiimid unter Zusatz von 3-Hydroxy-4-oxo-3,4-dihydro-1,2,3-benzotriazin (DCC/HOObt-Methode; Lit.: Chem. Ber. 103 (1970) 2034)

3. Dicyclohexylcarbodiimid unter Zusatz von N-Hydroxysuccinimid (DCC/HONSu-Methode; Lit.: Z. Naturforsch. 21b (1966) 426)

4. Alkanphosphonsäureanhydrid, wie n-Propylphosphonsäureanhydrid (PPA-Methode; Lit.: Angew. Chemie. Int. Ed. 19 (1980) 133)

5. Dialkylphosphinsäureanhydrid, wie Methylethylphosphinsäureanhydrid (MEPA-Methode; Lit.: US-Patent 4 426 325).

Als geeignete Lösungsmittel verwendet man bei dem erfindungsgemäßen Verfahren aus Gründen der Löslichkeit meist polare Lösungsmittel wie z.B. Dimethylacetamid, Dimethylformamid, Dimethylsulfoxid, Phosphorsäure-tris(dimethylamid), N-Methylpyrrolidon, Wasser oder Mischungen der genannten Lösungsmittel mit Wasser. Letzteres trifft vor allem beim MEPA-Verfahren zu. Aber auch Choroform,

Methylenchlorid oder Essigester kommen zum Einsatz. Die Synthese kann zwischen -10 und ca. 50 ° C, vorzugsweise -10 ° C und Raumtemperatur durchgeführt werden. Vorzugsweise beginnt man bei ca. 0 ° C und erhöht später auf Raumtemperatur.

Die Kondensation einer Carbonsäure der Formel IVa bzw. Va mit einem Alkohol der Formel IVb bzw. Vb zu einem Carbonsäureester läßt sich vorteilhaft nach der Dicyclohexylcarbodiimid(DCC)-Methode - wie in Angew. Chem. 90, 556 (1978) beschrieben - in einem inerten Lösungsmittel wie DMF unter Katalyse von 4-Dimethylaminopyridin im Temperaturbereich zwischen -20 bis +40 ° C, vorzugsweise -20 ° C bis Raumtemperatur durchführen.

Methoden, die zur Herstellung einer modifizierten Peptidbindung geeignet sind, werden in Janssen Chimica Acta, Vol. 3, Nr. 2 [1985] beschrieben. Vorzugsweise werden die folgenden Methoden herangezogen:

Synthese von Derivaten mit reduzierten Peptidbindungen durch reduktive Aminierung von Aldehyden mit Aminosäureestern mittels Natriumcyanoborhydrid (vgl. Borch et al., J. Amer Chem. Soc 93 [1971] 2897; 91 [1969] 3996) oder durch N-Alkylierung von Aminen oder Aminosäurederivativen (vgl. Houben-Weyl, Methoden der Org. Chemie, Bd. 11/1), indem man.

d) eine Verbindung der Formel VIa, VIb oder VIc mit einer Verbindung der Formel IIIb,

A'-CHO (VIa)

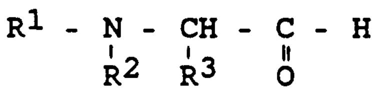

(VIb)

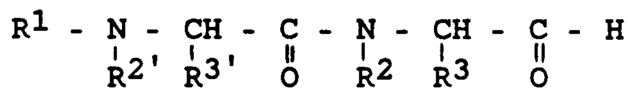

(VIc)

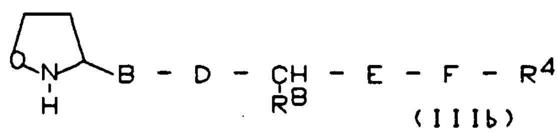

(IIIb)

in welchen B, D, E, F, $R^1$, $R^2$, $R^{2'}$, $R^3$, $R^{3'}$, $R^4$ und $R^8$ wie oben definiert sind und A' für ($C_1$-$C_8$)-Alkyl steht, das gegebenenfalls wie A unter $a_1$) substituiert ist, oder für ($C_3$-$C_8$)-Cycloalkyl, ($C_6$-$C_{12}$)-Aryl oder ($C_3$-$C_9$)-Heteroaryl steht, die gegebenenfalls wie A unter $a_2$) substituiert sind, umsetzt,

$e_1$) eine Verbindung der Formel VIIa mit einer Verbindung der Formel IVb,

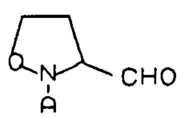

(VIIa)

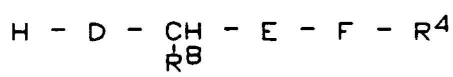

(IVb)

in welchen A, E, F, $R^4$ und $R^8$ wie oben definiert sind, A zusätzlich für [2,3:5,6]-Diisopropylidenmannofuranosyl und D für Imino oder N-Methylimino steht, umsetzt,

$e_2$) eine Verbindung der Formel VIIIa mit einer Verbindung der Formel VIIIb

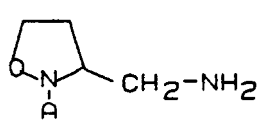

(VIIIa)

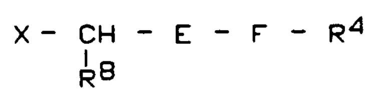

(VIIIb)

in welchen A, E, F, $R^4$ und $R^8$ wie oben definiert sind, wobei A zusätzlich H bedeuten kann und X für eine nucleophil ablösbare Abgangsgruppe wie OH, Cl, Br, I, Tosyl oder Triflat steht, umsetzt oder

$e_3$) analog dem unter b) (S. 9) beschriebenen Verfahren eine Verbindung der Formel IVa mit einer

Verbindung der Formel IVb umsetzt,

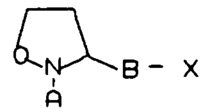

( IVa )

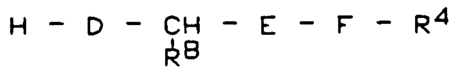

( IVb )

in welchen A, E, F, $R^4$ und $R^8$ wie oben definiert sind, B für $-CH_2-$, D für Imino, N-Methylimino oder Oxy und X für eine nucleophil ablösbare Abgangsgruppe wie OH, Cl, Br, I, Tosyl oder Triflat steht.

Man führt die reduktive Aminierung zweckmäßig in einem niederen Alkohol mit bis zu 4 C-Atomen wie Methanol bei einer Temperatur zwischen 0 ° C und dem Siedepunkt des Reaktionsgemisches, vorzugsweise zwischen 10 und 40 ° C und einem pH-Wert von 4 - 5 durch.

Die Synthese von Ketomethylen-Analogen der Formel I, in welchen B = CO und D $-CH_2-$ bedeuten, erfolgt durch Grignard- oder Beformatsky-Reaktion, indem man

f) die Komponenten IXa und IXb,

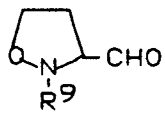

( IVa )

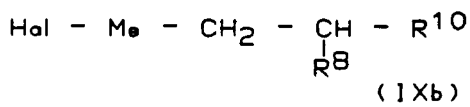

( IXb )

in welchen $R^8$ vorzugsweise H, $R^9$ vorzugsweise Trityl oder [2,3:5,6]Diisopropylidenmannofuranosyl, Me Mg oder Zn und $R^{10}$ eine unter den Bedingungen der Grignard-Reaktion stabile geschützte Carboxylfunktion, wie Oxazolinyl- oder 2,6,7-Trioxabicyclo[2,2,2]octanyl bedeuten, vorzugsweise in einem Ether wie Diethylether, Dibutylether oder Tetrahydrofuran bei einer Temperatur zwischen -80 ° C und dem Siedepunkt des Reaktionsgemisches umsetzt. Der resultierende Alkohol kann durch verschiedene literaturbekannte Oxidationsverfahren zum Keton oxidert und die Schutzgruppe $R^9$ säurekatalysiert entfernt werden. Die Kondensationsreaktionen nach den auf den Seiten 8 und 9 beschriebene Verfahren a) und c) liefern letzlich die Ketomethylenverbindungen der Formel I.

Endothiopeptide werden vorteilhaft durch Umsetzung der Verbindungen des allgemeinen Typs I mit Laweson's Reagenz (2,4-Bis-(4-methoxyphenyl)-1,3-dithia-2,4-diphosphetan-2,4-disulfid) erhalten (vgl. Synthesis 1979, 941).

Zum temporären Schutz weiterer funktioneller Gruppen sind Schutzgruppen geeignet, wie sie in der Peptidsynthese üblicherweise verwendet werden und beispielsweise in Kontakte Merck 3/79, Seiten 14 - 22 und 1/80, Seiten 23 - 35 beschrieben sind.

Urethanschutzgruppen der Aminofunktion sind beispielsweise Pyoc, Fmoc, Fcboc, Z, Boc, Ddz, Bpoc, $Z-(NO_2)$, Dobz, Moc, Mboc, Iboc, Adoc, Adpoc, Msc oder Pioc; bevorzugt sind Z oder Boc. Die Entfernung dieser Aminoschutzgruppen erfolgt mit Säuren, Basen oder reduktiv.

Schutzgruppen der Guanidinogruppe sind z.B. $NO_2$, Tosyl, Boc, Z, Mesitylen-2-sulfonyl (Mts) u.ä.. Die Abspaltung kann hydrolytisch oder hydrogenolytisch erfolgen.

Die COOH-Seitenfuntionen werden als Alkylester, vorzugsweise Methyl, Ethyl- oder tert. Butylester oder als Benzylester oder modifizierte Benzylester ($p-NO_2$, p-Cl, p-Br u.a.) blockiert. Die Deblockierung erfolgt durch alkalische oder saure Verseifung oder Hydrierung.

Hydroxylschutzgruppen sind beispielsweise tert. Butyl oder Benzyl.

Die erfindungsgemäßen Substanzen sind als irreversible Hemmstoffe der Prolylhydroxylase wirksam. Infolgedessen bewirken sie eine selektive Hemmung der kollagenspezifischen Hydroxylierungsreaktion, in dessen Verlauf Protein gebundenes Prolin durch das Enzym Prolylhydroxylase hydroxyliert wird. Bei Unterbindung dieser Reaktion mittels eines Inhibitors entsteht ein nichtfunktionsfähiges, unterhydroxylierts Kollagenmolekül, das von der Zelle nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens. Inhibitoren der Prolyhydroxylase sind deshalb geeignete Werkzeuge in der Therapie von Erkrankungen, in denen die Ablagerung von Kollagenen maßgeblich zum Krankheitsbild

beiträgt. Hierzu gehören u.a. Fibrosen der Lunge, Leber und Haut (Skleroderma) sowie die Atherosklerose.

Es ist weiterhin bekannt, daß Inhibitoren der Kollagenproduktion Antitumor- Eigenschaften besitzen. Durch die Reduktion der Kollagen-Synthese und -Ablagerung wird die für das Tumorwachstum notwendige Stromaumgestaltung beeinflußt (H. Dvorak, N. Engl. J. of Med. 315 (1986) 1650); und die Inhibitoren der Basalmembranbildung sind geeignet, das Wachstum verschiedener Tumore zu unterdrücken (W. Klohs et al. J. N. C. I. 75 (1985) 353).

Außerdem ist bekannt, daß die Inhibierung der Prolylhydroxylase durch bekannte Inhibitoren wie α,α-Dipyridyl zu einer Hemmung der C1q-Biosynthese von Makrophagen führt (W. Müller et al., FEBS Lett. 90, 218f (1978)). Dadurch kommt es zu einem Ausfall des klassischen Weges der Komplementaktivierung. Inhibitoren der Prolylhydroxylase wirken daher auch als Immunsuppressiva, z.B. bei Immunkomplexkrankheiten.

Die erfindungsgemäßen Substanzen können daher als Fibrosuppressiva, Immunsuppressiva, Antiatherosklerotika und in der Tumortherapie eingesetzt werden.

Die Hemmwirkung kann in einem Enzymtest analog der Methode von B. Peterkofsky und R. Di Blasio, Anal. Biochem. 66, 279 - 286 (1975) bestimmt werden. Dabei wird unterhydroxyliertes Kollagen mit Prolylhydroxylase in Gegenwart von Eisen(II)ionen, α-Ketoglutarat und Ascorbat enzymatisch hydroxyliert.

Zur Bestimmung des Suizidcharakters der Hemmung wird das Enzym in Gegenwart von Eisen(II)ionen, α-Ketoglutarat und Ascorbat mit den Hemmstoffen für unterschiedliche Zeiten vorinkubiert und anschließend in Gegenwart von Peptidsubstrat die Pestaktivität des Enzyms bestimmt. Zur Aktivitätsbestimmung können sowohl die oben beschriebene Methode nach Peterkovsky und Di Blasio als auch andere Methoden wie bei K.I. Kivirikko und R. Myllylä, Meth. Enzym. 82, 245 - 304 (1982) beschrieben, eingesetzt werden.

In Tabelle 1 ist die Hemmwirkung einiger ausgewählter erfindungsgemäßer Verbindungen aufgeführt. Angegeben ist die Konzentration, die nach einer Stunde 50 % des Enzyms inaktiviert.

**Tabelle 1:**

| Verbindung | $ID_{50}$ [60 min; μMol/l] |
|---|---|
| Ac-Pro-Opr-Gly-OtBu | 8000,0 |
| Ac-Pro-Opr-Gly-OBzl | 60,0 |
| Z-Ala-Opr-Gly-OtBu | 40,0 |
| Z-Ala-Opr-Gly-OH | 330,0 |
| Ac-Pro-Opr-Gly-NH-$CH_2$-$CH_2$-$C_6H_5$ | 4900,0 |
| Ac-Pro-Opr-Gly-$N(C_2H_5)_2$ | 16900,0 |
| Ac-Pro-Opr-Gly-NH-$CH_2$-$C_6H_5$ | 6000,0 |
| Z-Ala-Opr-Gly-OBzl | 3,0 |
| Z-(D)-Ala-Opr-Gly-OBzl | > 10000,0 |
| Z-Phe-Opr-Gly-OBzl | 0,8 |
| Msc-Glu(OBzl)-Opr-Gly-OBzl | 30,0 |
| Z-Val-Opr-Gly-OBzl | 40,0 |
| Z-Glu-Opr-Gly-OBzl | 5000,0 |
| $C_6H_5$-$CH_2$-CO-Opr-Gly-OBzl | 1000,0 |
| $C_6H_5$-$CH_2$-CO-Opr-Gly-O-$CH_2$-$C_6H_4$-4-$NO_2$ | 540,0 |
| Z-Phe-Opr-NH-$CH_2$-CO-$CH_2$-$CH_2$-$C_6H_5$ | 3,4 |
| $C_6H_5$-$CH_2$-$CH_2$-CO-Opr-Gly-OBzl | 2000,0 |
| Z-Tyr-Opr-Gly-OBzl | 2,4 |
| Z-Phe-Opr-Sar-OBzl | > 10000,0 |

Fortsetzung Tabelle 1:

| | |
|---|---|
| Z-Lys(Pht)-Opr-Gly-OBzl | 11,0 |
| Z-Phe-Opr-NH-$CH_2$-CO-$C_6H_5$ | 2,4 |
| $C_6H_5$-$CH_2$-Opr-Gly-OBzl | 610,0 |
| Z-Phe(r)-Opr-Gly-OBzl | 1,1 |
| H-Phe-Opr-NH-$CH_2$-CO-$C_6H_4$-4-Cl | 15,5 |
| Z-Phe-Opr-NH-$CH_2$-CO-$C_6H_4$-4-CN | 1,1 |
| Z-Phe-Opr-Gly-O-$CH_2$-$C_6H_4$-4-F | 0,8 |
| Z-Phe-Opr-Gly-O-$CH_2$-$CH_2$-$C_6H_5$ | 0,9 |
| Z-Tic-Opr-Gly-OBzl | 1,5 |

Die Hemmwirkung kann auch in der Zell- oder Gewebekultur bestimmt werden. Dazu können Fibroblasten oder andere Kollagen produzierende Zellen bzw. Calvarien oder andere Kollagen produzierende Organe eingsetzt werden. In Tabelle 2 ist die Hemmwirkung einiger erfindungsgemäßen Substanzen in der Calvarienkultur zusammengestellt. Angegeben ist die Konzentration, die zu einer 50 %igen Absenkung des Hydroxyprolin/Prolin-Quotienten bei Verfütterung von $^{14}$C-Prolin führt.

Tabelle 2:

| Verbindung | $IC_{50}$ [µM] |
|---|---|
| Z-Ala-Opr-Gly-OBzl | 1650 |
| Ac-Pro-Opr-Gly-OtBu | 7500 |
| Z-Val-Opr-Gly-OBzl | 380 |
| Z-Pro-Opr-Gly-OtBu | 550 |
| $C_6H_5$-$CH_2$-CO-Opr-Gly-OBzl | 2000 |
| Z-Ala-Opr-Gly-OBzl | 2500 |
| $C_6H_5$-$CH_2$-CO-Opr-Gly-$OCH_2$-$C_6H_4$-4-$NO_2$ | 2500 |

Die antifibrotische Wirkung kann im Modell der Tetrachlorkohlenstoff-induzierten Leberfibrose bestimmt werden. Dazu werden Ratten mit $CCl_4$ (1 ml/kg) gelöst in Olivenöl zweimal wöchentlich behandelt. Die Prüfsubstanz wird täglich, gegebenenfalls zweimal täglich per os oder intraperitoneal - gelöst in einem geeigneten verträglichen Lösungsmittel - verabreicht. Das Ausmaß der Leberfibrose wird histologisch bestimmt; der Anteil Kollagen in der Leber per Hydroxyprolinbestimmung - wie bei Kivirikko et al. (Anal. Biochem. 19, 249 f, (1967)) beschrieben - analysiert. Die Aktivität der Fibrogenese kann durch radioimmunologische Bestimmung von Killagenfragmenten und Prokollagenpeptiden im Serum bestimmt werden. Die erfindungsgemäßen Verbindungen sind in diesem Modell in Konzentration von 1 -100 mg/kg wirksam. Ein anderes Modell zur Evaluierung der antifibrotischen Wirking ist das der Bleomycin-induzierten Lungenfibrose wie bei Kelley et al. (J. Lab. Clin. Med. 96, 954, (1980)) beschrieben. Für die Evaluierung der Wirkung der erfindungsgemäßen Verbindungen auf Granulationsgewebe kann das Modell des Wattebauschgranuloms, wie bei Meier et al., Experientia 6, 469 (1950) beschrieben, herangezogen werden.

Die Erfindung betrifft daher eine Verbindung der Formel I zur Verwendung als Inhibitor der Prolylhydroxylase sowie zur Verwendung als Heilmittel bei Säugern und beim Menschen, insbesondere als Fibrosuppressiva, Immunsuppressiva, Antiatherosklerotika und in der Tumortherapie.

Die Erfindung betrifft weiterhin pharmazeutische Zubereitungen, die eine wirksame Menge einer

Verbindung der Formel I und einen physiologisch unbedenklichen Träger enthalten sowie ein Verfahren zur Herstellung dieser Zubereitungen, das dadurch gekennzeichnet ist, daß man den Wirkstoff zusammen mit dem Träger und gegebenenfalls weiteren Hilfs- oder Zusatzstoffen in eine geeignete Darreichungsform bringt. Der Wirkstoffanteil in diesen Zubereitungen beträgt in der Regel 0,1 bis 96 %.

Die Anwendung kann intranasal, buccal, intravenös, subcutan oder peroral erfolgen. Die Dosierung des Wirkstoffes hängt von der Warmblüter-Spezies, dem Körpergewicht, Alter und von der Applikationsart ab.

Die pharmazeutischen Präparte der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragées, Steckkapseln, wäßrige, alkoholische oder ölige Suspensionen oder wäßrige, alkoholische oder ölige Lösungen. Als inerte Träger kommen z.B. Gummi arabicum, Magnesiumcarbonat, Kaliumphosphat, Milchzucker, Glucose oder Stärke, insbesondere Maisstärke in Betracht. Dabei kann die Zubereitung sowohl als Trocken- als auch als Feuchtgranulat erfolgen. Als ölige Trägerstoffe oder Lösungsmittel kommen beispielsweise pflanzliche oder tierische Öle in Betracht, wie Sonnenblumenöl oder Lebertran.

Zur subsutanen oder intravenösen Applikation werden die aktiven Verbindungen oder deren physiologisch verträglichen Salze, gewünschtenfalls mit den dafür üblichen Substanzen wie Lösungsvermittler oder weiteren Hilfsstoffen, in Lösung, Suspension oder Emulsion gebracht. Dazu kommen z.B. in Frage: Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die folgenden Beispiele dienen zur Illustration der vorliegenden Erfindung, ohne daß diese darauf beschränkt wäre.

Verzeichnis der verwendeten Abkürzungen:

| | |
|---|---|
| AA | Aminosäureanalyse |
| Ac | Acetyl |
| Boc | tert.-Butoxycarbonyl |
| DC | Dünnschichtchromatographie |
| DCC | Dicyclohexylcarbodiimid |
| DCH | Dicyclohexylharnstoff |
| DMF | Dimethylformamid |
| DMSO | Dimethylsulfoxid |
| EE | Essigsäureethylester |
| FAB | Fast atom bombardment |
| Fmoc | 9-Fluorenylmethyloxycarbonyl |
| HOBt | 1-Hydroxybenzotriazol |
| HPhe | Homophenylalanin |
| M | Molekularpeak |
| MeOH | Methanol |
| MS | Massenspektrum |
| CHN | Elementaranalyse |
| NEM | N-Ethylmorpholin |
| tBu | tert. Butyl |
| Pht | Phthalyl |
| Schmp. | Schmelzpunkt |
| THF | Tetrahydrofuran |
| Z | Benzyloxycarbonyl |
| h | Stunde |
| min | Minute |
| RT | Raumtemperatur |
| Tic | 1,2,3,4-Tetrahydroisochinolin |
| Phg | Phenylglycin |

Die sonstigen für Aminosäuren und Schutzgruppen verwendeten Abkürzungen entsprechen dem in der Peptidchemie üblichen Buchstaben-Code, wie er z.B. in Europ. J. Biochem. 138, 9 - 37 (1984) beschrieben ist. Falls nicht ausdrücklich anders angegeben, handelt es sich immer um Aminosäuren der L-Konfiguration.

Die Abkürzung Opr wird in Einvernehmung mit dem üblichen Drei-Buchstaben-Code für die unnatürliche Aminosäure 5-Oxaprolin ( = Isoxazolidin-3-carbonsäure) eingeführt.

Opr, H-Opr-OH, Opr(r) und Opr(t) entsprechen somit folgenden Strukturen

Opr

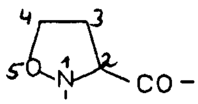

H-Opr-OH

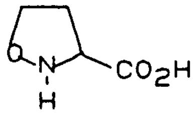

Opr(r)

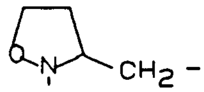

Opr(t)

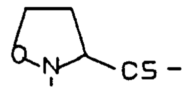

Die Synthese von H-Opr-OtBu erfolgt analog der Literaturvorschrift in J.C.S. Chem. Commun. 1981, 97 - 99.

| Chromatographie-Laufmittelsysteme: | |
|---|---|
| 1. $CHCl_3$/MeOH | 9 : 1 |
| 2. EE/Petrolether | 2 : 1 |
| 3. EE/Petrolether | 1 : 1 |
| 4. EE/Petrolether | 4 : 1 |
| 5. EE/Petrolether | 3 : 1 |
| 6. $CHCl_3$/$CH_3OH$ | 11 : 1 |
| 7. $CHCl_3$/$CH_3OH$/EE | 10 : 2 : 1 |

**Beispiele:**

A.1. Z-Phe-Opr-Gly-OBzl

A.1.1. Z-Phe-Opr-OtBu

600 mg des Hydrochlorids von H-Opr-OtBu, 860 mg Z-Phe-OH, 0,36 ml NEM und 395 mg HOBt werden in DMF gelöst. Nach Zugabe von 590 mg DCC läßt man 28 h bei Raumtemperatur reagieren. Das Lösungsmittel wird eingedampft, der Rückstand in 20 ml EE gelöst und je 3 mal mit 20 ml 20 %iger wäßriger Citronensäure und anschließend mit 20 ml gesättigter wäßriger Natriumhydrogencarbonatlösung ausgeschüttelt. Die organische Phase wird über Natriumsulfat getrocknet, filtriert und das Lösungsmittel abgedampft. Es werden 1,20 g Z-Phe-Opr-OtBu als farbloses Pulver isoliert. Das Rohprodukt wird ohne Reinigung direkt dem nächsten Reaktionsschritt (A.1.2.) unterworfen.

A.1.2. Z-Phe-Opr-OH

1,20 g Z-Phe-Opr-OtBu werden in 10 ml Trilfuoressigsäure gelöst. Man rührt 1 h bei RT, dampft das Lösungsmittel im Hochvakuum ab und chromatographiert den Rückstand über Kieselgel (System 7). Man isoliert 800 mg Z-Phe-Opr-OH;
$R_f$ (System 7) = 0,2; MS (FAB) : 399 (M + 1).

A.1.3. Z-Phe-Opr-Gly-OBzl

800 mg Z-Phe-Opr-OH, 640 mg des Hydrochlorids von H-Gly-OBzl, 260 µl NEM, 270 mg HOBt und 415 mg DCC werden in 20 ml DMF gelöst und 18 h bei RT gerührt. Man dampft das Lösungsmittel im Vakuum ab. Der Rüskstand wird an Kieselgel chromatographiert (System 3). Man isoliert 520 mg Z-Phe-Opr-Gly-OBzl als amorphen Feststoff.
$R_f$ (System 3) = 0,39; MS (FAB) : 546 (M + 1).

Analog der in Beispiel A.1.1. beschriebenen Verfahrensweise werden die Verbindungen der Beispiele A.2. bis A.62. durch Peptidkupplung synthetisiert, wobei zuerst eine Aminosäure N-terminal an H-Opr-OtBu geknüpft wird, anschließend der Ester hydrolysiert und die zweite Aminosäure addiert wird.

Beispiel Nr.

A.2. Z-Ala-Opr-Gly-OBzl
A.3. Z-(D)-Ala-Opr-Gly-OBzl
A.4. Z-Ala-Opr-Gly-OtBU
A.5. Z-Ala-Opr-Gly-OH
A.6. Z-Arg-Opr-Gly-OBzl
A.7. Z-Arg($Z_2$)-Opr-Gly-OBzl
A.8. Z-Asp-Opr-Gly-OBzl
A.9. Z-Asn-Opr-Gly-OBzl
A.10. Msc-Asn-Opr-Gly-OBzl
A.11 Z-Dab-Opr-Gly-OBzl
A.12. Z-Cys(Acm)-Opr-Gly-OBzl
A.13. Z-Cys(Bzl)-Opr-Gly-OBzl
A.14. Z-Cys-Opr-Gly-OBzl
A.15. Z-Gln-Opr-Gly-OBzl
A.16. Z-Gln-Opr-Gly-O-$CH_2$-$C_6H_4$ (p-$NO_2$)
A.17. Z-Glu(OBzl)-Opr-Gly-OBzl
A.18. Z-Glu-Opr-Gly-OBzl
A.19. Z-Gly-Opr-Gly-OBzl
A.20. Z-His-Opr-Gly-OBzl
A.21. Z-Ile-Opr-Gly-OBzl
A.22. Z-Leu-Opr-Gly-OBzl
A.23. Msc-Leu-Opr-Gly-OBzl
A.24. Z-Lys(Msc)-Opr-Gly-OBzl
A.25. Z-Lys(Pht)-Opr-Gly-OBzl
A.26. Z-Lys-Opr-Gly-OBzl
A.27. Z-Orn-Opr-Gly-OBzl
A.28. H-Phe-Opr-Gly-OBzl
A.29. Z-Phe-Opr-Gly-O-$CH_2$-$C_6H_4$-4-F
A.30. Z-Phe-Opr-(D)-Ala-OBzl
A.31. Z-Phe-Opr-Sar-OBzl
A.32. Ac-Pro-Opr-Gly-OBzl
A.33. Z-Pro-Opr-Gly-OBzl
A.34. Ac-Pro-Opr-Gly-OtBu
A.35. Z-Pro-Opr-Gly-OtBu
A.36. Z-Opr-Opr-Gly-OBzl
A.37. Z-Opr-Opr-Gly-O-$CH_2$-$C_6H_4$-4-Cl
A.38. Z-Ser-Opr-Gly-OBzl
A.39. Z-Thr(Bzl)-Opr-Gly-OBzl
A.40. Z-Thr-Opr-Gly-OBzl
A.41. Z-Tyr($CH_3$)-Opr-Gly-OBzl
A.42. Z-Tyr-Opr-Gly-OBzl
A.43. Z-Trp-Opr-Gly-OBzl
A.44. Z-Val-Opr-Gly-OBzl
A.45. Z-Phe-Opr-Gly-O-$CH_2$-$CH_2$-$C_6H_5$
A.46. Z-Tic-Opr-Gly-OBzl
A.47. Z-Phe-Opr-Gly-OEt
A.48. Z-Phe-Opr-Gly-(O-$CH_2$-$CH_2$)$_3$-$OCH_3$
A.49. Z-Phe-Opr-Gly-O-CH($C_6H_5$)$_2$
A.50. Z-Phe-Opr-Gly-O-$CH_2$-($C_5H_4N$)
A.51. Z-Phe-Opr-Gly-O-$CH_2$-$C_6H_4$-2-F
A.52. Z-Phe-Opr-Gly-O-$CH_2$-$C_6H_4$-3-F
A.53. Z-Phe-Opr-Gly-O-$CH_2$-$C_6H_4$-4-F
A.54. Z-Phe-Opr-Gly-O-($CH_2$)$_3$-$C_6H_5$
A.55. Z-Phe-Opr-Gly-O-($CH_2$)$_2$-$C_6H_4$-4-F
A.56. Z-(4′-Cl)Phe-Opr-Gly-OBzl
A.57. Z-(4′-F)Phe-Opr-Gly-OBzl

A.58. Z-(4'-$NO_2$)Phe-Opr-Gly-OBzl
A.59. Z-Phe-Opr-Gly-O-$CH_2$-CH($CO_2$Et)-$CH_2$-$C_6H_5$
A.60. Z-Phe-Opr-Gly-O-$CH_2$-CH(COOH)-$CH_2$-$C_6H_5$
A.61. Z-Phg-OPr-Gly-OBzl
A.62. Z-HPhe-Opr-Gly-OBzl

Um die Strukturen der so hergestellten Peptide zu bestätigen, wurden verschiedenartige analytische und spektroskopische Methoden angewandt. Einige Ergebnisse sind in Tabelle 3 zusammengefaßt.

**Tabelle 3 (Analyt. Daten der Tripeptide A-Opr-B (Beispiele A.1. bis A.62.))**

$^1$H-NMR : δ [ppm] =

| Nr. | 2-H(Opr) | 3-$H_2$(Opr) | 4-$H_2$(Opr) | 2-H(A) | 3-$H_x$(A) | 2-$H_y$(B) | Sonst. |
|---|---|---|---|---|---|---|---|
| A.1. | 4,85 | 2,5 -2,7 | 3,95-4,2 | 5,1 | 3,1 | 3,95 | MS; CHN |
| A.2. | 4,75 | 2,55-4,2 | 3,95-4,2 | 4,85 | 1,45 | 4,05 | MS; CHN |
| A.3. | 4,8 | 2,55-2,8 | 3,9 -4,05 | 4,2 | 1,35 | 3,95 | MS; CHN |
| A.4. | 4,75 | 2,55-2,9 | 3,9 -4,1 | 4,8 | 1,45 | 4,0 | MS; CHN |
| A.5. | 4,75 | 2,5 -2,75 | 3,9 -4,1 | 4,8 | 1,45 | 4,05 | MS |
| A.6. | 4,8 | 2,5 -2,75 | 3,95-4,1 | 4,85 | 1,75 | 4,0 | MS |
| A.7. | 4,8 | 2,5 -2,75 | 3,9 -4,1 | 4,75 | 1,75 | 4,0 | MS; CHN |
| A.8. | 4,8 | 2,55-2,7 | 3,9 -4,15 | 4,9 | 2,8 | 3,9 | MS |
| A.9. | 4,75 | 2,55-2,7 | 3,9 -4,1 | 4,85 | 2,7 | 3,95 | MS |

| Nr. | 2-H(Opr) | 3-$H_2$(Opr) | 4-$H_2$(Opr) | 2-H(A) | 3-$H_x$(A) | 2-$H_y$(B) | Sonst. |
|---|---|---|---|---|---|---|---|
| A.10. | 4,9 | 2,55-2,7 | 3,9 -4,1 | 4,9 | 2,65 | 4,0 | MS; CHN |
| A.11. | 4,9 | 2,5 -2,75 | 3,95-4,1 | 4,9 | 2,1 | 3,95 | MS |
| A.12. | 4,8 | 2,55-1,8 | 3,9 -4,15 | 4,95 | 3,1 | 4,0 | MS |
| A.13. | 4,85 | 2,55-2,75 | 3,9 -4,1 | 4,95 | 3,0 | 3,95 | MS |
| A.14. | 4,85 | 2,55-2,75 | 3,9 -4,05 | 5,0 | 3,05 | 3,95 | MS; CHN |
| A.15. | 4,85 | 2,5 -2,75 | 3,95-4,1 | 4,9 | 2,45 | 3,95 | MS; CHN |
| A.16. | 4,85 | 2,5 -2,75 | 3,95-4,1 | 4,9 | 2,5 | 4,0 | MS |
| A.17. | 4,7 | 2,5 -2,7 | 3,9 -4,1 | 4,85 | 2,2 | 4,0 | MS; CHN |
| A.18. | 4,85 | 2,55-2,7 | 3,9 -4,15 | 4,8 | 2,2 | 4,05 | MS; CHN |
| A.19. | 4,95 | 2,5 -2,7 | 3,9 -4,1 | 4,1 | - | 4,0 | MS; CHN |
| A.20. | 4,9 | 2,5 -2,75 | 3,9 -4,15 | 4,9 | 3,2 | 3,95 | MS |
| A.21. | 4,9 | 2,55-2,7 | 3,95-4,1 | 4,85 | 2,0 | 4,0 | MS |
| A.22. | 4,9 | 2,5 -2,85 | 4,0 -4,05 | 4,9 | 1,95 | 4,05 | MS |
| A.23. | 4,8 | 2,50-2,8 | 3,95-4,1 | 4,95 | 1,9 | 4,0 | MS |
| A.24. | 4,85 | 2,55-2.8 | 3,95-4,2 | 4,8 | 1,8 | 4,1 | MS; CHN |
| A.25. | 4,75 | 2,5 -2,75 | 3,9 -4,05 | 4,9 | 1,85 | 4,05 | MS |
| A.26. | 4,9 | 2,55-2,75 | 3,85-4,15 | 4,9 | 1,85 | 4,05 | MS; CHN |
| A.27. | 4,9 | 2,5 -2,75 | 3,9 -4,05 | 4,85 | 2,05 | 4,0 | MS |
| A.28. | 4,85 | 2,55-2,80 | 3,9 -4,1 | 4,25 | 2,95 | 3,95 | MS |
| A.29. | 4,85 | 2,5 -2,75 | 3,9 -4,15 | 5,0 | 3,1 | 3,95 | MS; CHN |
| A.30. | 5,05 | 2,5 -2,7 | 3,9 -4,2 | 4,8 | 3,1 | 4,55 | MS; CHN |
| A.31. | 4,95 | 2,5 -2,65 | 4,0 -4,3 | 5,1 | 3,1 | 3,75; 4,6 | MS; CHN |
| A.32. | 4,95 | 2,55-2,65 | 4,05-4,15 | 5,0 | 2,1 | 4,05 | MS; CHN |
| A.33. | 4,9 | 2,5 -2,6 | 3,6 -4,0 | 4,9 | 2,1 | 3,95 | MS; CHN |
| A.34. | 4,9 | 2,55-2,7 | 3,8 -3,85 | 4,85 | 2,1 | 4,1 | MS; CHN |
| A.35. | 4,9 | 2,5 -2,65 | 3,65-4,0 | 5,05 | 2,1 | 4,0 | MS; CHN |
| A.36. | 4,9 | 2,5 -2,7 | 3,75-3,95 | 5,0 | 2,6 | 4,1 | MS |
| A.37. | 4,8 | 2,55-2,7 | 3,7 -4,0 | 5,0 | 2,55 | 4,0 | MS |
| A.38. | 4,9 | 2,5 -2,7 | 3,75-4,05 | 5,0 | 2,75 | 4,0 | MS |
| A.39. | 4,95 | 2,5 -2,75 | 3,9 -4,15 | 5,05 | 3,9 | 4,0 | MS |
| A.40. | 4,95 | 2,5 -2,7 | 3,9 -4,1 | 4,85 | 3,9 | 4,0 | MS; CHN |
| A.41. | 4,8 | 2,5 -2,7 | 3,9 -4,15 | 4,9 | 3,1 | 3,95 | MS |
| A.42. | 4,85 | 2,5 -2,7 | 3,9 -4,15 | 4,9 | 3,0 | 4,05 | MS; CHN |
| A.43. | 4,95 | 2,5 -2,65 | 3,9 -4,1 | 4,95 | 3,0 | 4,05 | MS; CHN |
| A.44. | 4,7 | 2,55-2,7 | 3,9 -4,1 | 4,9 | 2,1 | 4,05 | MS; CHN |

| Nr. | 2-H(Opr) | 3-$H_2$(Opr) | 4-$H_2$(Opr) | 2-H(A) | 3-$H_x$(A) | 2-$H_y$(B) | Sonst. |
|---|---|---|---|---|---|---|---|
| A.45. | 4,85 | 2,5 -2,7 | 3,9 -4,15 | 5,0 | 3,0 | 4,05 | MS; CHN |
| A.46. | 4,8 | 2,5 -2,75 | 3,85-4,1 | 5,1 | 3,0-3,25 | 3,95 | MS; CHN |
| A.47. | 4,8 | 2,5 -2,8 | 3,9 -4,2 | 4,8 | 3,0-3,3 | 3,9 | MS; CHN |
| A.48. | 4,85 | 2,5 -2,85 | 3,9 -4,25 | 5,1 | 3,0-3,2 | 4,0 | MS; CHN |
| A.49. | 4,85 | 2,5 -2,8 | 3,85-4,2 | 5,05 | 3,1 | 3,9-4,15 | MS; CHN |
| A.50. | 4,85 | 2,5 -2,8 | 3,9 -4,15 | 5,1 | 3,1 | 4,05 | MS; CHN |
| A.51. | 4,85 | 2,5 -2,8 | 3,9 -4,15 | 5,05 | 3,1 | 3,95 | MS; CHN |
| A.52. | 4,8 | 2,5 -2,75 | 3,9 -4,2 | 5,05 | 3,1 | 3,95 | MS; CHN |
| A.53. | 4,8 | 2,5 -2,8 | 3,9 -4,2 | 5,1 | 3,1 | 3,95 | MS; CHN |
| A.54. | 4,85 | 2,45-2,8 | 3,9 -4,2 | 5,1 | 3,0-3,2 | 3,9 | MS; CHN |
| A.55. | 4,8 | 2,5 -2,8 | 3,9 -4,2 | 5,1 | 3,0-3,2 | 3,95 | MS; CHN |
| A.56. | 4,8 | 2,5 -2,9 | 3,9 -4,25 | 5,1 | 3,0-3,3 | 4,0-4,2 | MS; CHN |
| A.57. | 4,8 | 2,5 -2,85 | 3,9 -4,25 | 5,1 | 3,05-3,3 | 4,0-4,2 | MS; CHN |
| A.58. | 4,8 | 2,5 -2,9 | 3,9 -4,3 | 5,1 | 3,1-3,35 | 4,0-4,2 | MS; CHN |
| A.59. | 4,8 | 2,45-2,8 | 3,85-4,2 | 5,1 | 3,1-3,3 | 4,0 | MS; CHN |
| A.60. | 4,8 | 2,5 -2,8 | 3,85-4,2 | 5,1 | 3,0-3,3 | 4,0 | MS; CHN |
| A.61. | 4,9 | 2,5 -2,8 | 3,9 -4,2 | 5,8 | - | 3,95 | MS; CHN |
| A.62. | 4,75 | 2,4 -2,8 | 3,8 -4,2 | 4,85 | 1,8-2,1 | 3,9-4,1 | MS; CHN |

B.1. N-Phenylacetyl-Opr-Gly-4-Nitrobenzylester

B.1.1. N-Phenylacetyl-Opr-OtBu

500 mg des Hydrochlorids von H-Opr-OtBu, 324 mg Phenylessigsäure, 320 mg HOBt und 300 µl NEM in 10 ml DMF werden mit 490 mg DCC versetzt und 48 h bei RT gerührt. Man filtriert den Harnstoff ab, dampft das Lösungsmittel im Vakuum ab, löst den Rückstand in Essigester und wäscht mit wäßriger Citronensäure und wäßriger Natriumhydrogencarbonatlösung. Nach dem Trocken über Natriumsulfat und Abziehen des Lösungsmittels isoliert man 600 mg der geringfügig verunreinigen Zielverbindung N-Phenylacetyl-Opr-OtBu.

B.1.2. N-Phenylacetyl-Opr-OH

600 mg des Rohproduktes aus B.1.1. werden in 5 ml Trifluoressigsäure gelöst und 1 h bei RT gerührt. Nach Einengen im Hochvakuum wird aus EE umkristallisiert. Man isoliert 250 mg N-Phenylacetyl-Opr-OH. $R_f$ (System 7) = 0,65; MS (FAB) : 235 (M+1).

B.1.3. N-Phenylacetyl-Opr-Gly-4-Nitrobenzylester

70 mg N-Phenylacetyl-Opr-OH, 87 mg des Hydrobromids von H-Gly-OBzl-$NO_2$, 40 µl NEM und 41 mg HOBt werden in DMF gelöst. Num gibt man 62 mg DCC zu und rührt 8 h bei RT. Nach Abdampfen des Lösungsmittels wird an Kieselgel (System 5) chromatographiert. $R_f$ (System 5) = 0,43; MS (FAB) : 428 (M+1).

Analog der in Beispiel B.1. beschriebenen Verfahrensweise werden die Verbindungen der Beispiele B.2. bis B.19. synthetisiert, wobei zuerst die Carbonsäure N-terminal an H-Opr-OtBu gekuppelt wird, anschließend der Ester verseift und der C-terminale Baustein angeknüpft wird.

Beispiel Nr.:

- B.2. N-Benzoyl-Opr-Gly-OBzl
- B.3. N-4-Chlorbenzoyl-Opr-Gly-OBzl
- B.4. N-2-Fluorbenzoyl-Opr-Gly-OBzl
- B.5. N-3-Hydroxyphenylacetyl-Opr-Gly-OBzl
- B.6. N-4-Fluorphenylacetyl-Opr-Gly-OBzl
- B.7. N-Benzyloxycarbonyl-Opr-Gly-OBzl
- B.8. N-3-Phenylpropionyl-Opr-Gly-OBzl
- B.9. N-Phenoxyacetyl-Opr-Gly-OBzl
- B.10. N-4-Phenylbutyryl-Opr-Gly-OBzl
- B.11. N-4,4-Di-p-Fluorphenylbutyryl-Opr-Gly-OBzl
- B.12. N-Diphenylacetyl-Opr-Gly-OBzl
- B.13. Pivaloyl-Opr-Gly-OBzl
- B.14. N-(3-Phenylpropionyl)-Opr-Gly-$OCH_2$-$C_6H_4$-4-$NO_2$
- B.15. 4-$CH_3$-$C_6H_4$-$SO_2$-Opr-Gly-OBzl
- B.16. N-Phentylacetyl-Opr-Gly-O-$CH(CH_3)_2$
- B.17. N-(2-Acetoxy-3-phenylpropionyl)-Opr-Gly-OBzl
- B.18. N-(2-Hydroxy-3-phenylpropionyl)-Opr-Gly-OBzl
- B.19. N-[$C_6H_5$-$(CH_2)_2$-CO-O-CH($CH_2$-$C_6H_5$)-CO]-Opr-Gly-OBzl

**Tabelle 4 (Analytische Daten der Dipeptide A-Opr-Gly-OR) (Beispiele B.2. - B.19.)**

$^1$H-NMR : δ [ppm] =

| Nr. | 2-H(Opr) | 3-$H_2$(Opr) | 4-$H_2$(Opr) | $CH_2$(Gly) | CHx-CO(A) | Sonst. |
|---|---|---|---|---|---|---|
| B.1. | 4,85 | 2,45-2,85 | 3,65-4,15 | 4,1 | 3,8 | MS; CHN |
| B.2. | 4,9 | 2,5 -2,8 | 3,65-4,1 | 4,0 | - | MS; CHN |
| B.3. | 4,95 | 2,55-2,8 | 3,65-4,05 | 4,05 | - | MS; CHN |
| B.4. | 4,9 | 2,5 -2,8 | 3,65-4,1 | 4,0 | - | MS; CHN |
| B.5. | 4,85 | 2,5 -2,8 | 3,65-4,1 | 4,05 | 3,75 | MS; CHN |
| B.6. | 4,9 | 2,5 -2,8 | 3,65-4,15 | 4,05 | 4,0 | MS; CHN |
| B.7. | 4,9 | 2,5 -2,8 | 3,65-4,1 | 4,0 | 5,2 | MS; CHN |
| B.8. | 4,85 | 2,45-2,7 | 3,55-4,1 | 4,05 | 2,7 | MS; CHN |
| B.9. | 4,9 | 2,5 -2,8 | 3,6 -4,1 | 4,05 | 4,7 | MS; CHN |
| B.10. | 4,9 | 2,5 -2,75 | 3,6 -4,1 | 4,0 | 2,7 | MS; CHN |
| B.11. | 4,9 | 2,5 -2,8 | 3,6 -4,1 | 4,05 | 2,65 | MS; CHN |
| B.12. | 4,85 | 2,55-1,85 | 3,65-4,05 | 4,05 | 5,05 | MS; CHN |
| B.13. | 4,9 | 2,5 -2,8 | 3,6 -4,05 | 4,0 | - | MS; CHN |
| B.14. | 4,85 | 2,45-2,75 | 3,6 -4,1 | 4,1 | 3,7 | MS; CHN |
| B.15. | 4,15 | 2,5 -2,75 | 3,7 -4,05 | 4,2 | - | MS; CHN |
| B.16. | 4,85 | 2,45-2,8 | 3,7 -4,1 | 4,1 | 3,8 | MS; CHN |
| B.17. | 4,9 | 2,5 -2,8 | 4,0 -4,25 | 4,1 | 5,6 | MS; CHN |
| B.18. | 4,95 | 2,5 -2,8 | 4,0 -4,2 | 4,0 | 4,0 | MS; CHN |
| B.19. | 4,9 | 2,5 -2,8 | 3,8 -4,1 | 4,0 | 5,5 | MS; CHN |

C.1. N-4-Fluorbenzyl-Opr-Gly-OBzl

C.1.1. N-4-Fluorbenzyl-Opr-OH

Zu 145 mg 4-Fluorbenzaldehyd und 150 mg des Hydrochlorids von H-Opr-OtBu in 20 ml trockenem Methanol werden 30 mg Natriumcyanoborhydrid addiert. Durch Zugabe von 0,01 N Methanolischer HCl wird der pH-Wert der Lösung auf 4,0 gehalten. Man rührt 6 h bei RT, engt anschließend im Vakuum bis fast zur Trockne ein, nimmt in 100 ml 2 N wäßriger HCl auf, erhitzt über 30 min auf 50 °C, läßt abkühlen und extrahiert mit EE. Nach Trocknen der organischen Lösung über Magnesiumsulfat wird abfiltriert, das Lösungsmittel im Vakuum eingedampft und der Rückstand an Kieselgel (System 7) chromatographiert. Es werden 160 mg N-4-Fluorbenzyl-Opr-OH als weißer Feststoff isoliert. $R_f$ (System 7) = 0,15; MS (FAB) : 226 (M + 1).

C.1.2. N-4-Fluorbenzyl-Opr-Gly-OBzl

160 mg N-4-Fluorbenzyl-Opr-OH, 105 mg H-Gly-OBzl, 100 µl NEM, 100 mg HOBt und 150 mg DCC werden in 10 ml wasserfreiem DMF gelöst und 24 h bei RT gerührt. Man engt im Vakuum ein, nimmt in 20 ml Methylenchlorid auf, filtriert vom DCH ab und engt erneut ein. Nach Chromatographie an Kieselgel (System 3) werden 180 mg N-4-Fluorbenzyl-Opr-Gly-OBzl isoliert. $R_f$ (System 3) = 0,45; MS (FAB) : 373 (M + 1).

Analog der in Beispiel C.1. beschriebenen Verfahrensweise werden die Verbindungen der Beispiele C.2. bis C.6. synthetisiert.

Beipiel Nr.

| | |
|---|---|
| C.2. | $C_6H_5$-$CH_2$-Opr-Gly-OBzl |
| C.3. | 4-Cl-$C_6H_4$-$CH_2$-Opr-Gly-OBzl |
| C.4. | 4-$CH_3$-$C_6H_4$-$CH_2$-Opr-Gly-OBzl |
| C.5. | 4-$CH_3O$-$C_6H_4$-$CH_2$-Opr-Gly-OBzl |
| C.6. | Z-Phe(r)-Opr-Gly-OBzl |

**Tabelle 5 (Analytische Daten der Dipeptide der Beispiele C.1. - C.6.)**

$^1$H-NMR : δ [ppm] =

| Nr. | 2-H(Opr) | 3-$H_2$(Opr) | 4-$H_2$(Opr) | $CH_2$(Gly) | Sonst. |
|---|---|---|---|---|---|
| C.1. | 4,1 | 2,55-2,8 | 3,85-4,05 | 4,05 | MS; CHN |
| C.2. | 4,1 | 2,5 -2,8 | 3,9 -4,1 | 4,05 | MS; CHN |
| C.3. | 4,05 | 2,5 -2,8 | 3,85-4,05 | 4,0 | MS |
| C.4. | 4,05 | 2,5 -2,8 | 3,9 -4,05 | 4,05 | MS |
| C.5. | 4,05 | 2,55-2,8 | 3,9 -4,1 | 4,05 | MS |
| C.6. | 4,05 | 2,5 -2,75 | 3,85-4,05 | 4,05 | MS; CHN |

D.1. Z-Phe-Opr-NH-$CH_2$-CO-$CH_2$-$CH_2$-$C_6H_5$

D.1.1. (D,L)-4-Phenyl-2-hydroxy-butylamin-(1)

6,70 g Dihydrozimtaldehyd werden in 20 ml wasserfreiem Ethanol gelöst und mit 10 ml Nitromethan

und 1 ml Tetramethylguanidin versetzt und 1 h bei RT gerührt. Man engt im Vakuum ein, nimmt in 200 ml Ethanol und 5 ml Eisessig auf und hydriert nach Zusatz von 10 g aktiviertem Raney-Nickel über 5 h bei 50 °C und 25 bar $H_2$-Druck. Nun filtriert man vom Katalysator ab, engt im Vakuum ein, nimmt in gesättigter wäßriger Natriumhydrogencarbonatlösung auf und entfernt unpolare Verunreinigungen durch Extraktion mit Diethylether. 3-maliges Extrahieren mit Tetrahydrofuran, Trocknen über Natriumsulfat und Eingengen im Vakuum liefert 5,75 g (D,L)-4-Phenyl-2-hydroxybutylamin-(1) als farbloses Öl.

D.1.2. Z-Phe-Opr-NH-$CH_2$-CHOH-$CH_2$-$CH_2$-$C_6H_5$

843 mg Z-Phe-Opr-OH, 700 mg 4-Phenyl-2-hydroxybutylamin-(1), 285 mg HOBt und 436 mg DCC werden in 10 ml DMF gelöst. Nach zugabe von 270 µl NEM läßt man 18 h bei RT reagieren. Das Lösungsmittel wird im Vakuum eingedampft, der Rückstand in Essigester gelöst und 3-mal mit wäßriger Citronensäurelösung sowie mit gesättigter wäßriger Natriumhydrogencarbonatlösung behandelt. Nach Trocknen der Lösung über Natriumsulfat wird abfiltriert und eingedampft. Man chromatographiert an Kieselgel (System 4) und isoliert 680 mg der Zielverbindung. $R_f$ (System 4) = 0,35; MS (FAB) : 546 (M + 1).

D.1.3. Z-Phe-Opr-NH-$CH_2$-CO-$CH_2$-$CH_2$-$C_6H_5$

200 mg Z-Phe-Opr-NH-$CH_2$-CHOH-$CH_2$-$CH_2$-$C_6H_5$ werden in 20 ml wasserfreiem Methylenchlorid gelöst und nach Zusatz von 800 mg Pyridiniumchlorochromat 6 h bei RT gerührt. Man engt im Vakuum ein und entfernt die Chromsalze und geringfügig entstehende Nebenverbindungen durch Chromatographie an Kieselgel (System 4). Man isoliert 128 mg des Ketons. $R_f$ (System 4) = 0,68; MS (FAB) : 544 (M + 1).

Analog der in Beispiel D.1.beschriebenen Verfahrensweise werden die Verbindungen D.2. bis D.28. hergestellt.

Beispiel Nr.

D.2. Z-Phe-Opr-NH-$CH_2$-CO-$C_6H_5$
D.3. Z-Phe-Opr-NH-$CH_2$-CO-$C_6H_4$-4-OH
D.4. Z-Phe-Opr-NH-$CH_2$-CO-$C_6H_4$-4-$OCH_3$
D.5. Z-Phe-Opr-NH-$CH_2$-CO-$CH_2$-$C_6H_5$
D.6. Z-Phe-Opr-NH-$CH_2$-CO-$CH_2$-$C_6H_4$-4-OH
D.7. Z-Phe-Opr-NH-$CH_2$-CO-$CH_2$-$CH_2$-$C_6H_4$-4-Cl
D.8. Z-Phe-Opr-NH-$CH_2$-CO-$CH_2$-$CH_2$-$CH_2$-$C_6H_5$
D.9. Z-Phe-Opr-NH-$CH_2$-CO-$C_6H_4$-4-$CH(CH_3)_2$
D.10. $C_6H_5$-CO-Opr-NH-$CH_2$-CO-$CH_2$-$C_6H_5$
D.11. Z-Phe-Opr-NH-$CH_2$-CO-$C_6H_4$-4-CN
D.12. H-Phe-Opr-NH-$CH_2$-CO-$C_6H_4$-4-Cl
D.13.

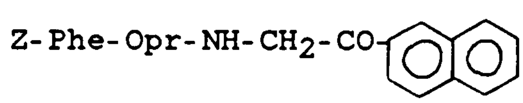

D.14. Z-Phe-Opr-NH-$CH_2$-CO-$C_6H_4$-4-Cl
D.15. Z-Phe-Opr-NH-$CH_2$-CO-$C_6H_4$-4-F
D.16. Z-Phe-Opr-NH-$CH_2$-CO-$C_6H_4$-4-$CF_3$
D.17. Z-Phe-Opr-NH-$CH_2$-CO-$C_6H_4$-4-COOH
D.18. Z-Phe-Opr-NH-$CH_2$-CO-$C_6H_4$-3-CN
D.19.

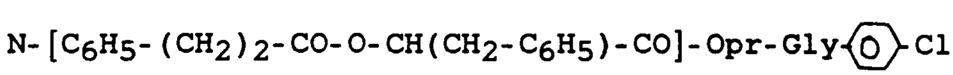

D.20. N-(2-Acetoxy-3-phenylpropionyl)-Opr-NH-$CH_2$-CO-$C_6H_4$-Cl
D.21. N-(2-Hydroxy-3-phenylpropionyl)-Opr-NH-$CH_2$-CO-$C_6H_4$-Cl
D.22. Z-Phe-Opr-NH-$CH_2$-CO-$C_6H_4$-4-$CO_2CH_3$
D.23. Boc-Phe-Opr-NH-$CH_2$-CO-$C_6H_4$-4-Cl

D.24. Z-Glu-Opr-NH-$CH_2$-CO-$C_6H_4$-4-Cl
D.25. Z-Cys-Opr-NH-$CH_2$-CO-$C_6H_4$-4-Cl
D.26. Z-Phe-Opr-NH-$CH_2$-CO-$CH_2$-CH($CO_2$Et)-$CH_2$-$C_6H_5$
D.27. $C_6H_5$-$(CH_2)_2$-$SO_2$-$CH_2$-CH($CH_2$-$C_6H_5$)-CO-Opr-NH-$CH_2$-CO-$C_6H_4$-4-CN
D.28. $C_6H_5$-$(CH_2)_2$-$SO_2$-$CH_2$-CH($CH_2$-$C_6H_5$)-CO-Opr-NH-$CH_2$-CO-$CH_2$-CH($CH_2$-$C_6H_5$)-$CO_2C_2H_5$

**Tabelle 6 (Analytische Daten der Dipeptide der Beispiele D.1. - D.28.)**

$^1$H-NMR : δ [ppm] =

| Nr. | 2-H(Opr) | 3-$H_2$(Opr) | 4-$H_2$(Opr) | N-$CH_2$-CO | Sonst. |
|---|---|---|---|---|---|
| D.1. | 4,9 | 2,55-2,7 | 3,95-4,2 | 4,0 | MS; CHN |
| D.2. | 5,1 | 2,6 -2,8 | 3,95-4,25 | 4,7 | MS; CHN |
| D.3. | 5,0 | 2,55-2,7 | 3,9 -4,2 | 4,6 | MS; CHN |
| D.4. | 4,9 | 2,5 -2,7 | 3,9 -4,15 | 4,6 | MS |
| D.5. | 5,0 | 2,55-2,7 | 3,9 -4,1 | 4,1 | MS; CHN |
| D.6. | 5,0 | 2,55-2,7 | 3,95-4,05 | 4,05 | MS |
| D.7. | 5,0 | 2,55-2,7 | 3,9 -4,15 | 4,1 | MS; CHN |
| D.8. | 4,9 | 2,55-2,7 | 3,9 -4,15 | 4,0 | MS; CHN |
| D.9. | 5,1 | 2,6 -2,8 | 3,95-4,2 | 4,7 | MS; CHN |
| D.10. | 4,9 | 2,5 -2,8 | 3,65-4,1 | 4,15 | MS; CHN |
| D.11. | 4,9 | 2,6 -2,8 | 3,95-4,25 | 4,7 | MS; CHN |
| D.12. | 4,95 | 2,5 -2,7 | 3,9 -4,15 | 4,65 | MS; CHN |

| Nr. | 2-H(Opr) | 3-$H_2$(Opr) | 4-$H_2$(Opr) | N-$CH_2$-CO | Sonst. |
|---|---|---|---|---|---|
| D.13. | 4,9 | 2,5 -2,85 | 3,9 -4,3 | 4,85 | MS; CHN |
| D.14. | 4,9 | 2,5 -2,7 | 3,95-4,3 | 4,6-4,8 | MS; CHN |
| D.15. | 4,95 | 2,55-2,85 | 3,9 -4,3 | 4,7 | MS; CHN |
| D.16. | 4,9 | 2,45-2,8 | 3,9 -4,3 | 4,7 | MS; CHN |
| D.17. | 4,9 | 2,50-2,85 | 3,9 -4,25 | 4,7 | MS; CHN |
| D.18. | 4,9 | 2,5 -2,85 | 3,9 -4,25 | 4,7 | MS; CHN |
| D.19. | 4,9 | 2,5 -2,75 | 3,9 -4,2 | 4,6-4,8 | MS; CHN |
| D.20. | 4,9 | 2,45-2,8 | 3,9 -4,25 | 4,7 | MS; CHN |
| D.21. | 4,95 | 2,5 -2,8 | 3,9 -4,3 | 4,6-4,8 | MS; CHN |
| D.22. | 4,9 | 2,5 -2,85 | 3,9 -4,3 | 4,7 | MS; CHN |
| D.23. | 4,9 | 2,5 -2,85 | 3,9 -4,3 | 4,65 | MS; CHN |
| D.24. | 4,75 | 2,5 -2,8 | 3,8 -4,25 | 4,55 | MS; CHN |
| D.25. | 4,95 | 2,55-2,85 | 3,9 -4,3 | 4,6-4,8 | MS; CHN |
| D.26. | 5,0 | 2,5 -2,75 | 3,9 -4,3 | 4,1 | MS; CHN |
| D.27. | 4,9 | 2,5 -2,8 | 3,9 -4,25 | 4,7 | MS; CHN |
| D.28. | 4,9 | 2,5 -2,75 | 3,9 -4,35 | 4,1 | MS; CHN |

E.1. Z-Phe-Opr(t)-Gly-OBzl

800 mg Z-Phe-Opr-Gly-OBzl werdenmit 350 mg Lawesson's Reagenz vermischt und in 10 ml wasserfreiem Benzol suspendiert. Beim Erhitzen auf 80 ° C wird die Lösung homogen. Man rührt 1 h bei dieser Temperatur, engt im Vakuum ein und chromatographiert den Rückstand an Kieselgel (System 3). Es werden 210 mg des Thioamids Z-Phe-Opr(t)-Gly-OBzl ($R_f$ (System 3) = 0,45; MS (FAB) : 562 (M + 1)) und 62 mg des Thioamids Z-Phe(t)-Opr-Gly-OBzl isoliert ($R_f$ (System 3) = 0,61; MS (FAB) : 562 (M + 1)).

Analog der in Beispiel E.1. beschriebenen Verfahrenzweise werden die Verbindungen E.2. und E.3. dargestellt.

Beispiel Nr.

E.2. Z-Phe-Opr(t)-NH-$CH_2$-CO-$CH_2$-$C_6H_5$

E.3. 4-F-$C_6H_4$-CO-Opr(t)-NH-$CH_2$-CO-$CH_2$-$C_6H_4$-4-Cl

Tabelle 7 (Analytische Daten der Dipeptide der Beispiele E.1. - E.3.)

$^1$H-NMR : δ [ppm] =

| Nr. | 2-H(Opr)(t) | 3-$H_2$(Opr(t)) | 4-$H_2$(Opr(t)) | N-$CH_2$-CO | Sonst. |
|---|---|---|---|---|---|
| E.1. | 5,2 | 2,55-2,75 | 3,95-4,2 | 4,1 | MS |
| E.2. | 5,1 | 2,5 -2,7 | 3,9 -4,25 | 4,15 | MS |
| E.3. | 5,0 | 2,55-2,1 | 3,75-4,1 | 4,05 | MS |

F. Die Synthese der Verbindungen des allgemeinen Strukturtyps A-Opr(r)-Gly-F-$R^4$ erfolgt in Abhängigkeit von den Resten A, F und $R^4$ nach einem der unter $e_1$), $e_2$) oder $e_3$) (Seite 11 f) beschriebenen Verfahren.

Beispiel Nr.

F.1. Z-Phe-Opr(r)-Gly-O-Bzl
F.2. Z-Phe-Opr(r)-Gly-O-$CH_2$-$C_6H_4$-4-Cl
F.3. Z-Phe-Opr(r)-NH-$CH_2$-CO-$C_6H_5$
F.4. Z-Phe-Opr(r)-NH-$CH_2$-CO-$C_6H_4$-4-OH
F.5. Z-Phe-Opr(r)-NH-$CH_2$-CO-$C_6H_4$-4-$OCH_3$
F.6. Z-Phe-Opr(r)-NH-$CH_2$-CO-$CH_2$-$C_6H_5$
F.7. Z-Phe-Opr(r)-NH-$CH_2$-CO-$CH_2$-$C_6H_4$-4-Cl
F.8. Z-NH-CH($C_6H_5$)-CO-Opr(r)-NH-$CH_2$-CO-$CH_2$-$C_6H_5$
F.9. $C_6H_5$-$CH_2$-$CH_2$-CO-O-CH($CH_2$-$C_6H_5$)-CO-Opr(r)-NH-$CH_2$-CO-$C_6H_4$-4-CN
F.10. $C_6H_5$-$CH_2$-$CH_2$-$SO_2$-$CH_2$-CH($CH_2$-$C_6H_5$)-CO-Opr(r)-NH-$CH_2$-CO-$C_6H_4$-4-Cl
F.11. $C_6$-$H_5$-$CH_2$-$CH_2$-SO-$CH_2$-CH($CH_2$-$C_6H_5$)-CO-Opr(r)-NH-$CH_2$-CO-$C_6H_4$-4-Cl
F.12. $C_6H_5$-$CH_2$-$CH_2$-$SO_2$-$CH_2$-CH($CH_2$-$C_6H_5$)-CO-Opr(r)-NH-$CH_2$-CO-$CH_2$-CH($CH_2$-$C_6H_5$)-$CO_2C_2H_5$
F.13. $C_6H_5$-$CH_2$-$CH_2$-$SO_2$-$CH_2$-CH($CH_2$-$C_6H_5$)-CO-Opr(r)-NH-$CH_2$-CO-$CH_2$-CH($CH_2$-$C_6H_5$)-COOH

G. Die Synthese von Ketomethylenanalogen der allgemeinen Formel A= Opr-$CH_2$-$CH_2$-CO-F-$R^4$ erfolgt nach dem auf Seite 13 beschriebenen Verfahren.

Beispiel Nr.

G.1. Z-Phe-Opr-$CH_2$-$CH_2$-CO-$CH_2$-$C_6H_5$
G.2. Z-Phe-Opr-$CH_2$-$CH_2$-CO-$C_6H_5$
G.3. Z-Phe-Opr-$CH_2$-$CH_2$-CO-$C_6H_4$-4-$OCH_3$
G.4. Z-Phe-Opr-$CH_2$-$CH_2$-CO-$CH_2$-$C_6H_4$-4-$OCH_3$
G.5. Z-Phe-Opr-$CH_2$-$CH_2$-CO-$CH_2$-$C_6H_4$
G.7. Z-NH-CH($C_6H_5$)-CO-Opr-$CH_2$-$CH_2$-CO-$CH_2$-$C_6H_5$
G.8. Z-Trp-Opr-$CH_2$-$CH_2$-CO-$CH_2$-$C_6H_5$

Tabelle 9 (Analytische Daten der Dipeptide der Beispiele G.1. - G.8.)

$^1$H-NMR : δ [ppm] =

| Nr. | 2-H(Opr) | 3-$H_2$(Opr) | 4-$H_2$(Opr) | Sonst. |
|---|---|---|---|---|
| G.1. | 5,2 | 2,55-2,75 | 3,9 -4,15 | MS; CHN |
| G.2. | 5,1 | 2,55-2,8 | 3,85-4,05 | MS |
| G.3. | 5,2 | 2,55-2,75 | 3,85-4,1 | MS |
| G.4. | 5,2 | 2,55-2,75 | 3,8 -4,05 | MS; CHN |
| G.5. | 5,1 | 2,55-2,8 | 3,8 -4,05 | MS |
| G.6. | 5,15 | 2,55-2,8 | 3,8 -4,1 | MS |
| G.7. | 5,2 | 2,55-2,75 | 3,9 -4,1 | MS |
| G.8. | 5,2 | 2,5 -2,8 | 3,8 -4,0 | MS |

**Patentansprüche**

**Patentansprüche für folgende Vertragsstaaten : AT, BE, CH, DE, FR, GB, IT, LI, LU, NL, SE**

**1.** Verbindung der Formel I

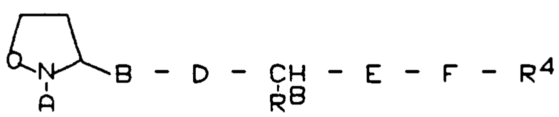

(I)

in welcher

A

a$_1$) ($C_1$-$C_8$)-Alkyl,
($C_1$-$C_8$)-Alkanoyl,
($C_1$-$C_8$)-Alkoxycarbonyl oder
($C_1$-$C_8$)-Alkylsulfonyl bedeutet,
in welchen jeweils 1, 2 oder 3 Wasserstoffatome gegebenenfalls durch 1, 2 oder 3 gleiche oder verschiedene Reste aus der Reihe
Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$)-Aryl-($C_1$-$C_5$)-alkyl ersetzt sind, oder in welchen jeweils 1 Wasserstoffatom gegebenenfalls durch einen Rest aus der Reihe

($C_3$-$C_8$)-Cycloalkyl,
($C_1$-$C_4$)-Alkylsulfonyl,
($C_1$-$C_4$)-Alkylsulfinyl,
($C_6$-$C_{12}$)-Aryl-($C_1$-$C_4$)-alkylsulfonyl,
($C_6$-$C_{12}$)-Aryl-($C_1$-$C_4$)-alkylsulfinyl,
($C_6$-$C_{12}$-Aryloxy,
($C_3$-$C_9$)-Heteroaryl und
($C_3$-$C_9$)-Heteroaryloxy
und
1 oder 2 Wasserstoffatome durch 1 oder 2 gleiche oder verschiedene Reste aus der Reihe
Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$-Aryl-($C_1$-$C_5$)-alkyl
ersetzt sind,
$a_2$) ($C_3$-$C_8$)-Cycloalkyl,
($C_6$-$C_{12}$)-Aryl,
($C_6$-$C_{12}$)-Arylsulfonyl oder
($C_3$-$C_9$)-Heteroaryl bedeutet,
wobei in den unter $a_1$) und $a_2$) definierten Resten jeweils ($C_6$-$C_{12}$)-Aryl bzw. ($C_3$-$C_9$)-Heteroaryl gegebenenfalls durch 1, 2 oder 3 gleiche oder verschiedene Reste aus der Reihe
Carboxy,
Amino,
Nitro,
($C_1$-$C_4$)-Alkylamino,
Hydroxy,
($C_1$-$C_4$)-Alkoxy,
Halogen,
Cyano,
Di-($C_1$-$C_4$)-alkylamino,
Carbamoyl,
Sulfamoyl und
($C_1$-$C_4$)-Alkoxycarbonyl
substituiert ist, oder
$a_3$) einen Rest der Formel IIa oder IIb bedeutet,

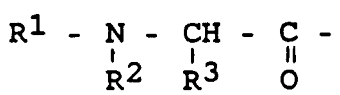

(IIa)

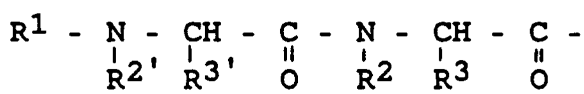

(IIb)

$R^1$
$b_1$) Wasserstoff bedeutet oder
$b_2$) wie A unter $a_1$) oder $a_2$) definiert ist,
$c_1$) $R^2$ und $R^{2'}$ gleich oder verschieden sind und Wasserstoff oder Methyl bedeuten,
$R^3$ und $R^{3'}$ gleich oder verschieden sind und Wasserstoff oder

$(C_1-C_6)$-Alkyl, das gegebenenfalls durch
Amino,
Benzyloxycarbonylamino,
Hydroxy,
Carboxy,
Carbamoyl,
Guanidino,
Ureido,
Mercapto,
Methylmercapto,
Phenyl,
4-Chlorphenyl,
4-Fluorphenyl,
4-Nitrophenyl,
4-Methoxyphenyl,
4-Hydroxyphenyl,
Phthalimido,
4-Imidazolyl,
3-Indolyl,
2-Thienyl,
3-Thienyl,
2-Pyridyl,
3-Pyridyl oder
Cyclohexyl monosubstituiert ist, bedeuten

$c_2$) $R^2$ und $R^3$ und/oder $R^{2'}$ und $R^{3'}$ jeweils zusammen für eine $[-CH_2-CH_2-CH_2-]$-Kette stehen, worin eine $CH_2$-Gruppe durch O ersetzt sein kann, oder

$c_3$) $R^2$ und $R^3$ und/oder $R^{2'}$ oder $R^{3'}$ jeweils zusammen für

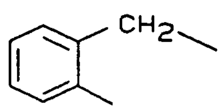

stehen,

B Carbonyl,
Thiocarbonyl,
Carbimidoyl,
$N-(C_1-C_3)$-Alkylcarbimidoyl,
$N-(C_1-C_3)$-Alkoxycarbimidoyl,
Sulfinyl,
$CR^5R^6$ oder
eine direkte Bindung bedeutet,

$d_1$) $R^5$ und $R^6$ gleich oder verschieden sind und Wasserstoff,
$(C_1-C_8)$-Alkyl oder
$(C_3-C_8)$-Cycloalkyl bedeuten oder

$d_2$) $R^5$ und $R^6$ gemeinsam für $-[CH_2]_m-$ mit $m = 4$ oder 5 stehen, worin 1 oder 2 $CH_2$-Gruppen gegebenenfalls durch 0, S und/oder $NR^7$ ersetzt sind,

D Imino,
N-Methylimino,
Oxy,
Methylen oder
eine direkte Bindung bedeutet,

E Carbonyl,
$C = NR^7$,
$C = N-OR^7$ oder
Sulfinyl bedeutet oder, falls F für eine bindung steht, auch Hydroxymethylen bedeuten kann,
$R^7$ wie $R^5$ unter $d_1$) definiert ist,

F Oxy,

Imino,
N-Methylimino oder
eine direkte Bindung bedeutet,
$R^4$ ($C_1$-$C_6$)-Alkyl,
($C_3$-$C_6$)-Cycloalkyl,
($C_6$-$C_{12}$)-Aryl,
($C_6$-$C_{12}$)-Aryl-($C_1$-$C_5$)-alkyl,
($C_6$-$C_{12}$)-Aryloxy-($C_1$-$C_5$)-alkyl,
($C_3$-$C_9$)-Heteroaryl-($C_1$-$C_5$)-alkyl bedeutet, worin jeweils Alkyl gegebenenfalls durch 1 oder 2 gleiche oder verschiedene Reste aus der Reihe
Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$)-Aryl-($C_1$-$C_5$)-alkyl substituiert ist, und worin jeweils ($C_6$-$C_{12}$)-Aryl bzw. ($C_3$-$C_9$)-Heteroaryl gegebenenfalls durch 1, 2 oder 3 gleiche oder verschiedene Reste aus der Reihe
Carboxy,
Cyano,
Amino,
Nitro,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl und
($C_1$-$C_4$)-Alkoxycarbonyl substituiert ist, und
$R^8$ wie $R^3$ unter $c_1$) definiert ist,

sowie deren physiologisch verträgliche Salze.

2. Verbindung der Formel I gemäß Anspruch 1, in welcher A gegebenenfalls substituiertes ($C_1$-$C_8$)-Alkyl, ($C_1$-$C_8$)-Alkanoyl oder gegebenenfalls substituiertes ($C_1$-$C_8$)-Alkoxycarbonyl bedeutet oder definiert ist, wie im Anspruch 1 unter $a_3$).

3. Verbindung der Formel I gemäß einem oder mehreren der Ansprüche 1 und 2, worin $R^8$ Wasserstoff bedeutet.

4. Verbindung der Formel I gemäß einem oder mehreren der Ansprüche 1 bis 3, worin B Carbonyl, Thiocarbonyl oder $CR^5R^6$ mit $R^5$ und $R^6$ = H bedeutet.

5. Verbindung der Formel I gemäß eine oder mehreren der Ansprüche 1 bis 4, worin D Imino, Oxy oder Methylen bedeutet.

6. Verbindung der Formel I gemäß einem oder mehreren der Ansprüche 1 bis 5, worin E Carbonyl bedeutet.

7. Verbindung der Formel I gemäß einem oder mehreren der Ansprüche 1 bis 6, worin F Oxy oder eine direkte Bindung bedeutet.

8. Verbindung der Formel I gemäß einem oder mehreren der Ansprüche 1 bis 7, worin $R^4$ gegebenenfalls

substituiertes ($C_6$-$C_{12}$)-Aryl oder gegebenenfalls im Alkylteil und/oder gegebenenfalls im Arylteil substituiertes ($C_6$-$C_{12}$)-Aryl-($C_1$-$C_5$)-alkyl bedeutet.

**9.** Verfahren zur Herstellung einer Verbindung der Formel I gemäß einem oder mehreren der Ansprüche 1 bis 8, dadurch gekennzeichnet, daß man die Verbindung in bekannter Weise aus den Fragmenten aufbaut, gegebenenfalls eine oder mehrere temporär eingeführte Schutzgruppe(n) abspaltet, Carbonylfunktionen gegebenenfalls in die Thia-Analogen umwandelt und die so erhaltene Verbindung der Formel I gegebenenfalls in ihr physiologisch verträgliches Salz überführt.

**10.** Verbindung gemäß einem oder mehreren der Ansprüche 1 bis 8 zur Verwendung als Inhibitor der Prolylhydroxylase.

**11.** Verbindung gemäß einem oder mehreren der Ansprüche 1 bis 8 zur Verwendung als Heilmittel.

**12.** Pharmazeutische Zubereitung enthaltend eine Verbindung gemäß einem oder mehreren der Ansprüche 1 bis 8 und einem physiologisch unbedenklichen Träger.

**Patentansprüche für folgende Vertragsstaaten : ES, GR**

**1.** Verfahren zur Herstellung einer Verbindung der Formel I

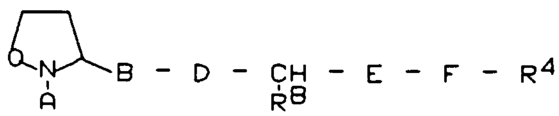

(I)

in welcher
A
a1) ($C_1$-$C_8$)-Alkyl,
($C_1$-$C_8$)-Alkanoyl,
($C_1$-$C_8$)-Alkoxycarbonyl oder
($C_1$-$C_8$)-Alkylsulfonyl bedeutet,
in welchen jeweils 1, 2 oder 3 Wasserstoffatome gegebenenfalls durch 1, 2 oder 3 gleiche oder verschiedene Reste aus der Reihe
Carboxy,
Amino,
($C_1$-$C_4$)-Alkylamino,
Hydroxy,
($C_1$-$C_4$)-Alkoxy,
Halogen,
Di-($C_1$-$C_4$)-alkylamino,
Carbamoyl,
Sulfamoyl,
($C_1$-$C_4$)-Alkoxycarbonyl,
($C_6$-$C_{12}$)-Aryl und
($C_6$-$C_{12}$)-Aryl-($C_1$-$C_5$)-alkyl ersetzt sind, oder in welchen jeweils 1 Wasserstoffatom gegebenenfalls durch einen Rest aus der Reihe
($C_3$-$C_8$)-Cycloalkyl,
($C_1$-$C_4$)-Alkylsulfonyl,
($C_1$-$C_4$)-Alkylsulfinyl,
($C_6$-$C_{12}$)-Aryl-($C_1$-$C_4$)-alkylsulfonyl,
($C_6$-$C_{12}$)-Aryl-($C_1$-$C_4$)-alkylsulfinyl,
($C_6$-$C_{12}$)-Aryloxy,
($C_3$-$C_9$)-Heteroaryl und
($C_3$-$C_9$)-Heteroaryloxy
und
1 oder 2 Wasserstoffatome durch 1 oder 2 gleiche oder verschiedene Reste aus der Reihe

Carboxy,
Amino,
$(C_1-C_4)$-Alkylamino,
Hydroxy,
$(C_1-C_4)$-Alkoxy,
Halogen,
Di-$(C_1-C_4)$-alkylamino,
Carbamoyl,
Sulfamoyl,
$(C_1-C_4)$-Alkoxycarbonyl,
$(C_6-C_{12})$-Aryl und
$(C_6-C_{12})$-Aryl-$(C_1-C_5)$-alkyl
ersetzt sind,
$a_2$) $(C_3-C_8)$-Cycloalkyl,
$(C_6-C_{12})$-Aryl,
$(C_6-C_{12})$-Arylsulfonyl oder
$(C_3-C_9)$-Heteroaryl bedeutet,
wobei in den unter $a_1$) und $a_2$) definierten Resten jeweils $(C_6-C_{12})$-Aryl bzw. $(C_3-C_9)$-Heteroaryl gegebenenfalls durch 1, 2 oder 3 gleiche oder verschiedene Reste aus der Reihe
Carboxy,
Amino,
Nitro,
$(C_1-C_4)$-Alkylamino,
Hydroxy,
$(C_1-C_4)$-Alkoxy,
Halogen,
Cyano,
Di-$(C_1-C_4)$-alkylamino,
Carbamoyl,
Sulfamoyl und
$(C_1-C_4)$-Alkoxycarbonyl
substituiert ist, oder
$a_3$) einen Rest der Formel IIa oder IIb bedeutet,

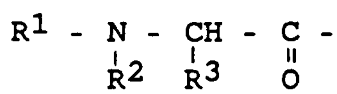

(IIa)

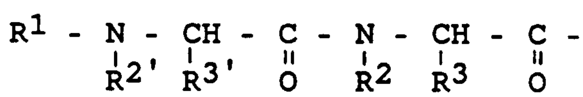

(IIb)

$R^1$
$b_1$) Wasserstoff bedeutet oder
$b_2$) wie A unter $a_1$) oder $a_2$) definiert ist,
$c_1$) $R^2$ und $R^{2'}$ gleich oder verschieden sind und Wasserstoff oder Methyl bedeuten,
$R^3$ und $R^{3'}$ gleich oder verschieden sind und Wasserstoff oder
$(C_1-C_6)$-Alkyl, das gegebenenfalls durch
Amino,
Benzyloxycarbonylamino,
Hydroxy,
Carboxy,
Carbamoyl,
Guanidino,
Ureido,
Mercapto,
Methylmercapto,
Phenyl,

4-Chlorphenyl,
4-Fluorphenyl,
4-Nitrophenyl,
4-Methoxyphenyl,
4-Hydroxyphenyl,
Phthalimido,
4-Imidazolyl,
3-Indolyl,
2-Thienyl,
3-Thienyl,
2-Pyridyl,
3-Pyridyl oder
Cyclohexyl monosubstituiert ist, bedeuten
$c_2$) $R^2$ und $R^3$ und/oder $R^{2'}$ und $R^{3'}$ jeweils zusammen für eine [-$CH_2$-$CH_2$-$CH_2$-]-Kette stehen, worin eine $CH_2$-Gruppe durch O ersetzt sein kann, oder
$c_3$) $R^2$ und $R^3$ und/oder $R^{2'}$ oder $R^{3'}$ jeweils zusammen für

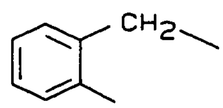

stehen,
B Carbonyl,
Thiocarbonyl,
Carbimidoyl,
N-($C_1$-$C_3$)-Alkylcarbimidoyl,
N-($C_1$-$C_3$)-Alkoxycarbimidoyl,
Sulfinyl,
$CR^5R^6$ oder
eine direkte Bindung bedeutet,
$d_1$) $R^5$ und $R^6$ gleich oder verschieden sind und Wasserstoff,
($C_1$-$C_8$)-Alkyl oder
($C_3$-$C_8$)-Cycloalkyl bedeuten oder
$d_2$) $R^5$ und $R^6$ gemeinsam für -$[CH_2]_m$- mit m = 4 oder 5 stehen, worin 1 oder 2 $CH_2$-Gruppen gegebenenfalls durch 0, S und/oder $NR^7$ ersetzt sind,
D Imino,
N-Methylimino,
Oxy,
Methylen oder
eine direkte Bindung bedeutet,
E Carbonyl,
$C = NR^7$,
$C = N\text{-}OR^7$ oder
Sulfinyl bedeutet oder, falls F für eine Bindung steht, auch Hydroxymethylen bedeuten kann,
$R^7$ wie $R^5$ unter $d_1$) definiert ist,
F Oxy,
Imino,
N-Methylimino oder
eine direkte Bindung bedeutet,
$R^4$ ($C_1$-$C_6$)-Alkyl,
($C_3$-$C_6$)-Cycloalkyl,
($C_6$-$C_{12}$)-Aryl,
($C_6$-$C_{12}$)-Aryl-($C_1$-$C_5$)-alkyl,
($C_6$-$C_{12}$)-Aryloxy-($C_1$-$C_5$)-alkyl,
($C_3$-$C_9$)-Heteroaryl-($C_1$-$C_5$)-alkyl bedeutet, worin jeweils Alkyl gegebenenfalls durch 1 oder 2 gleiche oder verschiedene Reste aus der Reihe
Carboxy,

Amino,
$(C_1-C_4)$-Alkylamino,
Hydroxy,
$(C_1-C_4)$-Alkoxy,
Halogen,
Di-$(C_1-C_4)$-alkylamino,
Carbamoyl,
Sulfamoyl,
$(C_1-C_4)$-Alkoxycarbonyl,
$(C_6-C_{12})$-Aryl und
$(C_6-C_{12})$-Aryl-$(C_1-C_5)$-alkyl substituiert sind, und worin jeweils $(C_6-C_{12})$-Aryl bzw. $(C_3-C_9)$-Heteroaryl gegebenenfalls durch 1, 2 oder 3 gleiche oder verschiedene Reste aus der Reihe
Carboxy,
Cyano,
Amino,
Nitro,
$(C_1-C_4)$-Alkylamino,
Hydroxy,
$(C_1-C_4)$-Alkoxy,
Halogen,
Di-$(C_1-C_4)$-alkylamino,
Carbamoyl,
Sulfamoyl und
$(C_1-C_4)$-Alkoxycarbonyl substituiert sind,
und
$R^8$ wie $R^3$ unter $c_1$) definiert ist,

sowie deren physiologisch verträgliche Salze, dadurch gekennzeichnet, daß man die Verbindung in bekannter Weise aus den Fragmenten aufbaut, gegebenenfalls eine oder mehrere temporär eingeführte Schutzgruppe(n) abspaltet, Carbonylfunktionen gegebenenfalls in die Thia-Analogen umwandelt und die so erhaltene Verbindung der Formel I gegebenenfalls in ihr physiologisch verträgliches Salz überführt.

**2.** Verfahren gemäß Anspruch 1, dadurch gekennzeichnet, daß man eine Verbindung der Formel I herstellt, in welcher A gegebenenfalls substituiertes $(C_1-C_8)$-Alkyl, $(C_1-C_8)$-Alkanoyl oder gegebenenfalls substituiertes $(C_1-C_8)$-Alkoxycarbonyl bedeutet oder definiert ist, wie im Anspruch 1 unter $a_3$).

**3.** Verfahren gemäß einem oder mehreren der Ansprüche 1 und 2, dadurch gekennzeichnet, daß man eine Verbindung der Formel I herstellt, worin $R^8$ Wasserstoff bedeutet.

**4.** Verfahren gemäß einem oder mehreren der Ansprüche 1 bis 3, dadurch gekennzeichnet, daß man eine Verbindung der Formel I herstellt, worin B Carbonyl, Thiocarbonyl oder $CR^5R^6$ mit $R^5$ und $R^6$ = H bedeutet.

**5.** Verfahren gemäß einem oder mehreren der Ansprüche 1 bis 4, dadurch gekennzeichnet, daß man eine Verbindung der Formel I herstellt, worin D Imino, Oxy oder Methylen bedeutet.

**6.** Verfahren gemäß einem oder mehreren der Ansprüche 1 bis 5, dadurch gekennzeichnet, daß man eine Verbindung der Formel I herstellt, worin E Carbonyl bedeutet.

**7.** Verfahren gemäß einem oder mehreren der Ansprüche 1 bis 6, dadurch gekennzeichnet, daß man eine Verbindung der Formel I herstellt, worin F Oxy oder eine direkte Bindung bedeutet.

**8.** Verfahren gemäß einem oder mehreren der Ansprüche 1 bis 7, dadurch gekennzeichnet, daß man eine Verbindung der Formel I herstellt, worin $R^4$ gegebenenfalls substituiertes $(C_6-C_{12})$-Aryl oder gegebenenfalls im Alkylteil und/oder gegebenenfalls im Arylteil substituiertes $(C_6-C_{12})$-Aryl-$(C_1-C_5)$-alkyl bedeutet.

**9.** Verfahren zur Herstellung einer pharmazeutischen Zubereitung, enthaltend eine Verbindung der Formel I gemäß einem der Ansprüche 1 bis 8, dadurch gekennzeichnet, daß man diese zusammen mit einem

physiologisch unbedenklichen Träger und gegebenenfalls weiterne Hilfs- oder Zusatzstoffen in eine geeignete Darreichungsform bringt.

**Claims**

**Claims for the following Contracting States : AT, BE, CH, DE, FR, GB, IT, LI, LU, NL, SE**

**1.** A compound of the formula I

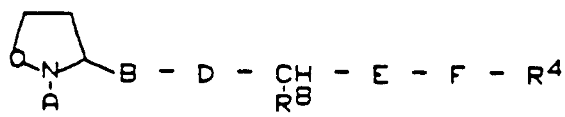

(I)

in which

A denotes

$a_1$) ($C_1$-$C_8$)-alkyl, ($C_1$-$C_8$)-alkanoyl,

($C_1$-$C_8$)-alkoxycarbonyl or ($C_1$-$C_8$)-alkylsulfonyl,

in each of which 1, 2 or 3 hydrogen atoms are optionally replaced by 1, 2 or 3 identical or different radicals from the series comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl, or in each of which 1 hydrogen atom is optionally replaced by a radical from the series comprising ($C_3$-$C_8$)-cycloalkyl, ($C_1$-$C_4$)-alkylsulfonyl, ($C_1$-$C_4$)-alkylsulfinyl, ($C_6$-$C_{12}$)-aryl-($C_1$-$C_4$)-alkylsulfonyl, ($C_6$-$C_{12}$)-aryl-($C_1$-$C_4$)-alkylsulfinyl, ($C_6$-$C_{12}$)-aryloxy, ($C_3$-$C_9$)-heteroaryl and ($C_3$-$C_9$)-heteroaryloxy, and 1 or 2 hydrogen atoms are replaced by 1 or 2 identical or different radicals from the series comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl,

denotes

$a_2$) ($C_3$-$C_8$)-cycloalkyl, ($C_6$-$C_{12}$)-aryl, ($C_6$-$C_{12}$)-arylsulfonyl or ($C_3$-$C_9$)-heteroaryl, each of ($C_6$-$C_{12}$)-aryl or ($C_3$-$C_9$)-heteroaryl in the radicals defined under $a_1$) and $a_2$) being optionally substituted by 1, 2 or 3 identical or different radicals from the series comprising carboxyl, amino, nitro, ($C_1$-$C_4$)-alkylamino, hydroxyl, ($C_1$-$C_4$)-alkoxy, halogen, cyano, di-($C_1$-$C_4$)-alkylamino, carbamoyl, sulfamoyl and ($C_1$-$C_4$)-alkoxycarbonyl, or

denotes

$a_3$) a radical of the formula IIa or IIb

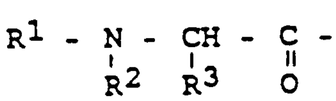

(IIa)

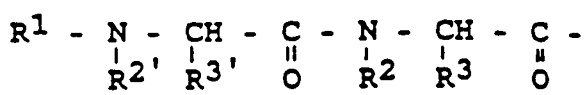

(IIb)

$R^1$

$b_1$) denotes hydrogen or

$b_2$) is defined as A under $a_1$) or $a_2$),

$c_1$) $R^2$ and $R^{2'}$ are identical or different and denote hydrogen or methyl,

$R^3$ and $R^{3'}$ are identical or different and denote hydrogen or ($C_1$-$C_6$)-alkyl which is optionally monosubstituted by amino, benzyloxycarbonylamino, hydroxyl, carboxyl, carbamoyl, guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pryridyl or cyclohexyl,

$c_2$) $R^2$ and $R^3$ and/or $R^{2'}$ and $R^{3'}$ in each case together represent a [-$CH_2$-$CH_2$-$CH_2$-] chain in which a $CH_2$ group can be replaced by O, or

$c_3$) $R^2$ and $R^3$ and/or $R^2$ and $R^{3'}$ in each case together represent

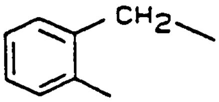

B denotes carbonyl, thiocarbonyl, carbimidoyl, N-($C_1$-$C_3$)-alkylcarbimidoyl, N-($C_1$-$C_3$)-alkoxycarbimidoyl, sulfinyl, $CR^5R^6$ or a direct bond,

d₁) $R^5$ and $R^6$ are identical or different and denote hydrogen, ($C_1$-$C_8$)-alkyl or ($C_3$-$C_8$)-cycloalkyl, or

d₂) $R^5$ and $R^6$ together represent -$[CH_2]_m$- with m = 4 or 5, in which 1 or 2 $CH_2$ groups are optionally replaced by O, S and/or $NR^7$,

D denotes imino, N-methylimino, oxy, methylene or a direct bond,

E denotes carbonyl, $C=NR^7$, $C=N\text{-}OR^7$ or sulfinyl, or, if F represents a bond, can also denote hydroxymethylene,

$R^7$ is defined as $R^5$ under $d_1$),

F denotes oxy, imino, N-methylimino or a direct bond,

$R^4$ denotes ($C_1$-$C_6$)-alkyl, ($C_3$-$C_6$)-cycloalkyl, ($C_6$-$C_{12}$)-aryl, ($C_6$-$C_{12}$)-aryl-($C_1$-$C_5$)-alkyl, ($C_6$-$C_{12}$)-aryloxy-($C_1$-$C_5$)-alkyl, ($C_3$-$C_9$)-heteroaryl-($C_1$-$C_5$)-alkyl, in which each alkyl is optionally substituted by 1 or 2 identical or different radicals from the series comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl, and in which each ($C_6$-$C_{12}$)-aryl or ($C_3$-$C_9$)-heteroaryl is optionally substituted by 1, 2 or 3 identical or different radicals from the series comprising carboxyl, cyano, amino, nitro, ($C_1$-$C_4$)-alkylamino, hydroxyl, ($C_1$-$C_4$)-alkoxy, halogen, di-($C_1$-$C_4$)-alkylamino, carbamoyl, sufamoyl and ($C_1$-$C_4$)-alkoxycarbonyl,

and

$R^8$ is defined as $R^3$ under $c_1$),

and its physiologically tolerated salts.

2. A compound of the formula I as claimed in claim 1, in which A denotes optionally substituted ($C_1$-$C_8$)-alkyl, ($C_1$-$C_8$)-alkanoyl or optionally substituted ($C_1$-$C_8$)-alkoxycarbonyl, or is defined as in claim 1 under $a_3$).

3. A compound of the formula I as claimed in one or more of claims 1 and 2, in which $R^8$ denotes hydrogen.

4. A compound of the formula I as claimed in one or more of claims 1 to 3, in which B denotes carbonyl, thiocarbonyl or $CR^5R^6$, with $R^5$ and $R^6 = H$.

5. A compound of the formula I as claimed in one or more of claims 1 to 4, in which D denotes imino, oxy or methylene.

6. A compound of the formula I as claimed in one or more of claims 1 to 5, in which E denotes carbonyl.

7. A compound of the formula I as claimed in one or more of claims 1 to 6, in which F denotes oxy or a direct bond.

8. A compound of the formula I as claimed in one or more of claims 1 to 7, in which $R^4$ denotes optionally substituted ($C_6$-$C_{12}$)-aryl or denotes ($C_6$-$C_{12}$)-aryl-($C_1$-$C_5$)-alkyl which is optionally substituted in the alkyl moiety and/or optionally substituted in the aryl moiety.

9. A process for the preparation of a compound of the formula I as claimed in one or more of claims 1 to 8, which comprises synthesis of the compound in a known manner from the fragments, where appropriate elimination of one or more temporarily introduced protective group(s), where appropriate conversion of carbonyl groups into the thia analogs, and where appropriate conversion of the resulting compound of the formula I into its physiologically tolerated salt.

10. A compound as claimed in one or more of claims 1 to 8 for use as an inhibitor of prolyl hydroxylase.

**11.** A compound as claimed in one or more of claims 1 to 8 for use as a medicine.

**12.** A pharmaceutical composition containing a compound as claimed in one or more of claims 1 to 8, and a physiologically acceptable vehicle.

**Claims for the following Contracting States : ES, GR**

**1.** A process for the preparation of a compound of the formula I

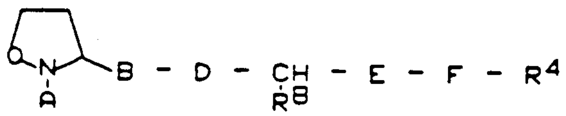

(I)

in which

A denotes

$a_1$) ($C_1$-$C_8$)-alkyl, ($C_1$-$C_8$)-alkanoyl,
($C_1$-$C_8$)-alkoxycarbonyl or ($C_1$-$C_8$)-alkylsulfonyl,
in each of which 1, 2 or 3 hydrogen atoms are optionally replaced by 1, 2 or 3 identical or different radicals from the series comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl, or in each of which 1 hydrogen atom is optionally replaced by a radical from the series comprising ($C_3$-$C_8$)-cycloalkyl, ($C_1$-$C_4$)-alkylsulfonyl, ($C_1$-$C_4$)-alkylsulfinyl, ($C_6$-$C_{12}$)-aryl-($C_1$-$C_4$)-alkylsulfonyl, ($C_6$-$C_{12}$)-aryl-($C_1$-$C_4$)-alkylsulfinyl, ($C_6$-$C_{12}$)-aryloxy, ($C_3$-$C_9$)-heteroaryl and ($C_3$-$C_9$)-heteroaryloxy, and 1 or 2 hydrogen atoms are replaced by 1 or 2 identical or different radicals from the series comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl,
denotes
$a_2$) ($C_3$-$C_8$)-cycloalkyl, ($C_6$-$C_{12}$)-aryl, ($C_6$-$C_{12}$)-arylsulfonyl or ($C_3$-$C_9$)-heteroaryl, each of ($C_6$-$C_{12}$)-aryl or ($C_3$-$C_9$)-heteroaryl in the radicals defined under $a_1$) and $a_2$) being optionally substituted by 1, 2 or 3 identical or different radicals from the series comprising carboxyl, amino, nitro, ($C_1$-$C_4$)-alkylamino, hydroxyl, ($C_1$-$C_4$)-alkoxy, halogen, cyano, di-($C_1$-$C_4$)-alkylamino, carbamoyl, sulfamolyl and ($C_1$-$C_4$)-alkoxycarbonyl, or
denotes
$a_3$) a radical of the formula IIa or IIb

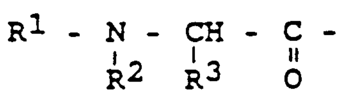

(IIa)

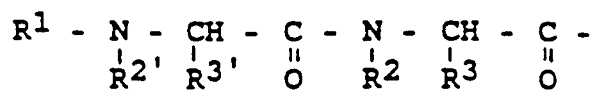

(IIb)

$R^1$

$b_1$) denotes hydrogen or
$b_2$) is defined as A under $a_1$) or $a_2$),
$c_1$) $R^2$ and $R^{2'}$ are identical or different and denote hydrogen or methyl,
$R^3$ and $R^{3'}$ are identical or different and denote hydrogen or ($C_1$-$C_6$)-alkyl which is optionally monosubstituted by amino, benzyloxycarbonylamino, hydroxyl, carboxyl, carbamoyl, guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pryridyl or cyclohexyl,
$c_2$) $R^2$ and $R^3$ and/or $R^{2'}$ and $R^{3'}$ in each case together represent a [-$CH_2$-$CH_2$-$CH_2$-] chain in which a $CH_2$ group can be replaced by O, or
$c_3$) $R^2$ and $R^3$ and/or $R^{2'}$ and $R^{3'}$ in each case together represent

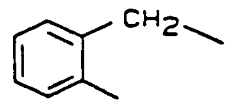

B denotes carbonyl, thiocarbonyl, carbimidoyl, N-($C_1$-$C_3$)-alkylcarbimidoyl, N-($C_1$-$C_3$)-alkoxycarbimidoyl, sulfinyl, $CR^5R^6$ or a direct bond,

$d_1$) $R^5$ and $R^6$ are identical or different and denote hydrogen, ($C_1$-$C_8$)-alkyl or ($C_3$-$C_8$)-cycloalkyl, or

$d_2$) $R^5$ and $R^6$ together represent $-[CH_2]_m-$ with m = 4 or 5, in which 1 or 2 $CH_2$ groups are optionally replaced by O, S and/or $NR^7$,

D denotes imino, N-methylimino, oxy, methylene or a direct bond,

E denotes carbonyl, $C=NR^7$, $C=N\text{-}OR^7$ or sulfinyl, or, if F represents a bond, can also denote hydroxymethylene,

$R^7$ is defined as $R^3$ under $d_1$),

F denotes oxy, imino, N-methylimino or a direct bond,

$R^4$ denotes ($C_1$-$C_6$)-alkyl, ($C_3$-$C_6$)-cycloalkyl, ($C_6$-$C_{12}$)-aryl, ($C_6$-$C_{12}$)-aryl-($C_1$-$C_5$)-alkyl, ($C_6$-$C_{12}$)-acyloxy-($C_1$-$C_5$)-alkyl, ($C_3$-$C_9$)-heteroaryl-($C_1$-$C_5$)-alkyl, in which each alkyl is optionally substituted by 1 or 2 identical or different radicals from the series comprising carboxyl, amino, ($C_1$-$C_4$)-alkylamino, hydroxyl, ($C_1$-$C_4$)-alkoxy, halogen, di-($C_1$-$C_4$)-alkylamino, carbamoyl, sulfamoyl, ($C_1$-$C_4$)-alkoxycarbonyl, ($C_6$-$C_{12}$)-aryl and ($C_6$-$C_{12}$)-aryl-($C_1$-$C_5$)-alkyl, and in which each ($C_6$-$C_{12}$)-aryl or ($C_3$-$C_9$)-heteroaryl is optionally substituted by 1, 2 or 3 identical or different radicals from the series comprising carboxyl, cyano, amino, nitro, ($C_1$-$C_4$)-alkylamino, hydroxyl, ($C_1$-$C_4$)-alkoxy, halogen, di-($C_1$-$C_4$)-alkylamino, carbamoyl, sufamoyl and ($C_1$-$C_4$)-alkoxycarbonyl,

and

$R^5$ is defined as $R^3$ under $c_1$),

and its physiologically tolerated salts which comprises synthesis of the compound in a known manner from the fragments, where appropriate elimination of one or more temporarily introduced protective group(s), where appropriate conversion of carbonyl groups into the thia analogs, and where appropriate conversion of the resulting compound of the formula I into its physiologically tolerated salt.

**2.** The process as claimed in claim 1, wherein a compound of the formula I is prepared in which A denotes optionally substituted ($C_1$-$C_8$)-alkyl, ($C_1$-$C_8$)-alkanoyl or optionally substituted ($C_1$-$C_8$)-alkoxycarbonyl, or is defined as in claim 1 under $a_3$).

**3.** The process as claimed in one or more of claims 1 and 2, wherein a compound of the formula I is prepared in which $R^8$ denotes hydrogen.

**4.** The process as claimed in one or more of claims 1 to 3, wherein a compound of the formula I is prepared in which B denotes carbonyl, thiocarbonyl or $CR^5R^6$, with $R^5$ and $R^6$ = H.

**5.** The process as claimed in one or more of claims 1 to 4, wherein a compound of the formula I is prepared in which D denotes imino, oxy or methylene.

**6.** The process as claimed in one or more of claims 1 to 5, wherein a compound of the formula I is prepared in which E denotes carbonyl.

**7.** The process as claimed in one or more of claims 1 to 6, wherein a compound of the formula I is prepared in which F denotes oxy or a direct bond.

**8.** The process as claimed in one or more of claims 1 to 7, wherein a compound of the formula I is prepared in which $R^4$ denotes optionally substituted ($C_6$-$C_{12}$)-aryl or denotes ($C_6$-$C_{12}$)-aryl-($C_1$-$C_5$)-alkyl which is optionally substituted in the alkyl moiety and/or optionally substituted in the aryl moiety.

**9.** A process for the preparation of a pharmaceutical composition containing a compound of the formula I as claimed in any of claims 1 to 8 , which comprises the latter being converted with a physiologically acceptable vehicle and, where appropriate, further auxiliaries or additives into a suitable form for administration.

**Revendications**

**Revendications pour les Etats contractants suivants : AT, BE, CH, DE, FR, GB, IT, LI, LU, NL, SE**

**1.** Composé de formule I

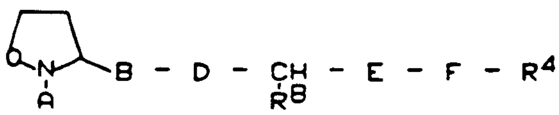 (I)

dans laquelle

A représente

$a_1$) alkyle en $C_{1-8}$, alcanoyle en $C_{1-8}$, alcoxy($C_{1-8}$)carbonyle ou alkylsulfonyle en $C_{1-8}$, groupes dans chacun desquels 1, 2 ou 3 atomes d'hydrogène peut (peuvent) être éventuellement remplacé(s) par 1, 2 ou 3 restes identiques ou différents choisis parmi carboxy, amino, alkyl($C_{1-4}$)amino, hydroxy, alcoxy en $C_{1-4}$, halogène, dialkyl($C_{1-4}$)amino, carbamoyle, sulfamoyle, alcoxy($C_{1-4}$)carbonyle, aryle en $C_{6-12}$ et aryl($C_{6-12}$)alkyle($C_{1-5}$), ou bien dans chacun desquels 1 atome d'hydrogène peut être éventuellement remplacé par un reste choisi parmi cycloalkyle en $C_{3-8}$, alkylsulfonyle en $C_{1-4}$, alkylsulfinyle en $C_{1-4}$, aryl($C_{6-12}$)alkyl($C_{1-4}$)sulfonyle, aryl($C_{6-12}$)alkyl($C_{1-4}$)sulfinyle, aryloxy en $C_{6-12}$, hétéroaryle en $C_{3-9}$ et hétéroaryloxy en $C_{3-9}$, et 1 ou 2 atome(s) d'hydrogène peut (peuvent) être remplacé(s) par 1 ou 2 restes identiques ou différents choisis parmi carboxy, amino, alkyl($C_{1-4}$)amino, hydroxy, alcoxy en $C_{1-4}$, halogène, dialkyl($C_{1-4}$)amino, carbamoyle, sulfamoyle, alcoxy($C_{1-4}$)carbonyle, aryle en $C_{6-12}$ et aryl($C_{6-12}$)alkyle($C_{1-5}$),

$a_2$) cycloalkyle en $C_{3-8}$, aryle en $C_{6-12}$, aryl($C_{6-12}$)sulfonyle ou hétéroaryle en $C_{3-9}$, chacun des restes aryle en $C_6$-$C_{12}$ ou hétéroaryle en $C_3$-$C_9$, respectivement définis dans $a_1$) et $a_2$) pouvant être substitué par 1, 2, ou 3 restes identiques ou différents choisis parmi carboxy, amino, nitro, alkyl($C_{1-4}$)amino, hydroxy, alcoxy en $C_{1-4}$, halogène, cyano, dialkyl($C_{1-4}$)amino, carbamoyle, sulfamoyle et alcoxy($C_{1-4}$)carbonyle, ou bien

$a_3$) un reste de formule IIa ou IIb:

$R_1$-N($R^2$)-CH($R^3$)-C(=O)- (IIa)

$R_1$-N($R^{2'}$)-CH($R^{3'}$)-C(=O)-N($R^2$)-CH($R^3$)-C(=O)- (IIb),

dans lesquelles $R^1$ représente

$b_1$) un atome d'hydrogène, ou

$b_2$) tel que défini à propos de A $a_1$) et $a_2$),

$c_1$) $R^2$ et $R^{2'}$, identiques ou différents, représentent un atome d'hydrogène ou un groupe méthyle, $R^3$ et $R^{3'}$, identiques ou différents, représentent un atome d'hydrogène ou un groupe alkyle en $C_{1-6}$,

éventuellement monosubstitué par amino, benzyloxycarbonylamino, hydroxy, carboxy, carbamoyle, guanidino, uréido, mercapto, méthylmercapto, phényle, 4-chlorophényle, 4-fluorophényle, 4-nitrophényle, 4-méthoxyphényle, 4-hydroxyphényle, phtalimido, 4-imidazolyle, 3-indolyle, 2-thiényle, 3-thiényle, 2-pyridyle, 3-pyridyle ou cyclohexyle,

$c_2$) les ensembles $R^2$ et $R^3$ et/ou $R^{2'}$ et $R^{3'}$ représentent chacun une chaîne [-$CH_2$-$CH_2$-$CH_2$-], dans laquelle un groupe $CH_2$ peut être remplacé par O, ou

$c_3$) les ensembles $R^2$ et $R^3$ et/ou $R^{2'}$ et $R^{3'}$ sont chacun

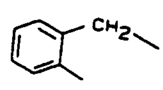

B représente un groupe carbonyle, thiocarbonyle, carbimidoyle, N-alkyl($C_{1-3}$)carbimidoyle, N-alcoxy($C_{1-3}$)carbimidoyle, sulfinyle, $CR^5R^6$ ou une liaison directe,

$d_1$) $R^5$ et $R^6$, identiques ou différents, représentent un atome d'hydrogène, un groupe alkyle en $C_{1-8}$ ou cycloalkyle en $C_{3-8}$, ou bien

$d_2$) $R^5$ et $R^6$ ensemble représentent une chaîne -$[CH_2]_m$- où m = 4 ou 5, un ou deux groupes

$CH_2$ étant éventuellement remplacé(s) par O, S et/ou $NR^7$,
D représente un groupe imino, N-méthylimino, oxy, méthylène ou une liaison directe,
E représente un groupe carbonyle, $C=NR^7$, $C=N\text{-}OR^7$ ou sulfinyle, ou bien, lorsque F est une liaison, il peut également représenter un groupe hydroxyméthylène,
$R^7$ est défini comme $R^5$ à propos de $d_1$),
F représente un groupe oxy, imino, N-méthylimino ou une liaison directe,
$R^4$ est un groupe alkyle en $C_{1-6}$, cycloalkyle en $C_{3-6}$, aryle en $C_{6-12}$, aryl($C_{6-12}$)-alkyle($C_{1-5}$), aryloxy($C_{6-12}$)alkyle($C_{1-5}$), ou hétéroaryl($C_{3-9}$)alkyle($C_{1-5}$), le groupe alkyle pouvant chaque fois être substitué par 1 ou 2 restes identiques ou différents choisis parmi carboxy, amino, alkyl($C_{1-4}$)-amino, hydroxy, alcoxy en $C_{1-4}$, halogène, dialkyl($C_{1-4}$)amino, carbamoyle, sulfamoyle, alcoxy-($C_{1-4}$)carbonyle, aryle en $C_{6-12}$ et
aryl($C_{6-12}$)alkyle($C_{1-5}$), et les groupes aryle en $C_{6-12}$ et hétéroaryle en $C_{3-9}$ pouvant être substitués chacun par 1, 2 ou 3 restes identiques ou différents choisis parmi carboxy, cyano, amino, nitro, alkyl($C_{1-4}$)amino, hydroxy, alcoxy en $C_{1-4}$, halogène, dialkyl($C_{1-4}$)amino, carbamoyle, sulfamoyle et alcoxy($C_{1-4}$)carbonyle, et
$R^8$ est défini comme $R^3$ à propos de $c_1$),
ainsi que leurs sels physiologiquement compatibles.

**2.** Composé de formule I selon la revendication 1, dans lequel A représente un groupe alkyle en $C_{1-8}$ éventuellement substitué, alcanoyle en $C_{1-8}$ ou alcoxy($C_{1-8}$)carbonyle éventuellement substitué, ou bien est défini comme dans la revendication 1 à propos de $a_3$).

**3.** Composé de formule I selon une ou plusieurs des revendications 1 et 2, dans lequel $R^8$ est un atome d'hydrogène.

**4.** Composé de formule I selon une ou plusieurs des revendications 1 à 3, dans lequel B représente un groupe carbonyle, thiocarbonyle ou $CR^5R^6$, où $R^5$ et $R^6$ sont H.

**5.** Composé de formule I selon une ou plusieurs des revendications 1 à 4, dans lequel D représente un groupe imino, oxy ou méthylène.

**6.** Composé de formule I selon une ou plusieurs des revendications 1 à 5, dans lequel E est un groupe carbonyle.

**7.** Composé de formule I selon une ou plusieurs des revendications 1 à 6, dans lequel F est oxy ou une liaison directe.

**8.** Composé de formule I selon une ou plusieurs des revendications 1 à 7, dans lequel $R^4$ représente un groupe aryle en $C_{6-12}$ éventuellement substitué ou un groupe aryl($C_{6-12}$)alkyle($C_{1-5}$) éventuellement substitué dans la partie alkyle et/ou éventuellement substitué dans la partie aryle.

**9.** Procédé de préparation d'un composé de formule I, selon une ou plusieurs des revendications 1 à 8, caractérisé en ce qu'on édifie le composé de manière connue à partir des fragments, on scinde éventuellement un ou plusieurs groupe(s) protecteur(s) temporairement introduit(s), on transforme éventuellement les fonctions carbonyle en analogues thia et on transforme éventuellement le composé de formule I ainsi obtenu en son sel physiologiquement compatible.

**10.** Composé selon une ou plusieurs des revendications 1 à 8, pour son utilisation comme inhibiteur de la prolylhydroxylase.

**11.** Composé selon une ou plusieurs des revendications 1 à 8, pour son utilisation comme médicament.

**12.** Composition pharmaceutique contenant un composé selon une ou plusieurs des revendications 1 à 8 et un véhicule physiologiquement acceptable.

**Revendications pour les Etats contractants suivants : ES, GR**

**1.** Procédé de préparation d'un composé de formule I

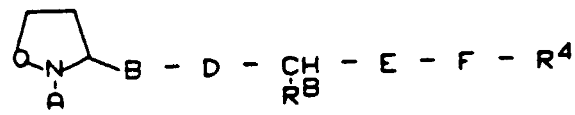 (I)

dans laquelle

A représente

$a_1$) alkyle en $C_{1-8}$, alcanoyle en $C_{1-8}$, alcoxy($C_{1-8}$)carbonyle ou alkylsulfonyle en $C_{1-8}$, groupes dans chacun desquels 1, 2 ou 3 atomes d'hydrogène peut (peuvent) être éventuellement remplacé(s) par 1, 2 ou 3 restes identiques ou différents choisis parmi carboxy, amino, alkyl-($C_{1-4}$)amino, hydroxy, alcoxy en $C_{1-4}$, halogène, dialkyl($C_{1-4}$)amino, carbamoyle, sulfamoyle, alcoxy($C_{1-4}$)carbonyle, aryle en $C_{6-12}$ et aryl($C_{6-12}$)alkyle($C_{1-5}$), ou bien dans chacun desquels 1 atome d'hydrogène peut être éventuellement remplacé par un reste choisi parmi cycloalkyle en $C_{3-8}$, alkylsulfonyle en $C_{1-4}$, alkylsulfinyle en $C_{1-4}$, aryl($C_{6-12}$)alkyl($C_{1-4}$)sulfonyle, aryl($C_{6-12}$)alkyl($C_{1-4}$)sulfinyle, aryloxy en $C_{6-12}$, hétéroaryle en $C_{3-9}$ et hétéroaryloxy en $C_{3-9}$, et 1 ou 2 atome(s) d'hydrogène peut (peuvent) être remplacé(s) par 1 ou 2 restes identiques ou différents choisis parmi carboxy, amino, alkyl-($C_{1-4}$)amino, hydroxy, alcoxy en $C_{1-4}$, halogène, dialkyl($C_{1-4}$)amino, carbamoyle, sulfamoyle, alcoxy($C_{1-4}$)carbonyle, aryle en $C_{6-12}$ et aryl($C_{6-12}$)alkyle($C_{1-5}$),

$a_2$) cycloalkyle en $C_{3-8}$, aryle en $C_{6-12}$, aryl($C_{6-12}$)sulfonyle ou hétéroaryle en $C_{3-9}$, chacun des restes aryle en $C_6$-$C_{12}$ ou hétéroaryle en en $C_3$-$C_9$, respectivement définis dans $a_1$) et $a_2$) pouvant être substitué par 1, 2 ou 3 restes identiques ou différents choisis parmi carboxy, amino, nitro, alkyl($C_{1-4}$)amino, hydroxy, alcoxy en $C_{1-4}$, halogène, cyano, dialkyl($C_{1-4}$)amino, carbamoyle, sulfamoyle et alcoxy($C_{1-4}$)carbonyle, ou bien

$a_3$) un reste de formule IIa ou IIb:

$R_1$-N($R^2$)-CH($R^3$)-C(=O)- (IIa)

$R_1$-N($R^{2'}$)-CH($R^{3'}$)-C(=O)-N($R^2$)-CH($R^3$)-C(=O)- (IIb),

dans lesquelles $R^1$ représente

$b_1$) un atome d'hydrogène, ou

$b_2$) tel que défini à propos de A $a_1$) et $a_2$),

$c_1$) $R^2$ et $R^{2'}$, identiques ou différents, représentent un atome d'hydrogène ou un groupe méthyle, $R^3$ et $R^{3'}$, identiques ou différents, représentent un atome d'hydrogène ou un groupe alkyle en $C_{1-6}$,

éventuellement monosubstitué par amino, benzyloxycarbonylamino, hydroxy, carboxy, carbamoyle, guanidino, uréido, mercapto, méthylmercapto, phényle, 4-chlorophényle, 4-fluorophényle, 4-nitrophényle, 4-méthoxyphényle, 4-hydroxyphényle, phtalimido, 4-imidazolyle, 3-indolyle, 2-thiényle, 3-thiényle, 2-pyridyle, 3-pyridyle ou cyclohexyle,

$c_2$) les ensembles $R^2$ et $R^3$ et/ou $R^{2'}$ et $R^{3'}$ représentent chacun une chaîne [-$CH_2$-$CH_2$-$CH_2$-], dans laquelle un groupe $CH_2$ peut être remplacé par O, ou

$c_3$) les ensembles $R^2$ et $R^3$ et/ou $R^{2'}$ et $R^{3'}$ sont chacun

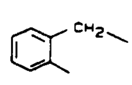

B représente un groupe carbonyle, thiocarbonyle, carbimidoyle, N-alkyl($C_{1-3}$)carbimidoyle, N-alcoxy($C_{1-3}$)carbimidoyle, sulfinyle, $CR^5R^6$ ou une liaison directe,

$d_1$) $R^5$ et $R^6$, identiques ou différents, représentent un atome d'hydrogène, un groupe alkyle en $C_{1-8}$ ou cycloalkyle en $C_{3-8}$, ou bien

$d_2$) $R^5$ et $R^6$ ensemble représentent une chaîne -$[CH_2]_m$- où m = 4 ou 5, un ou deux groupes $CH_2$ étant éventuellement remplacé(s) par O, S et/ou $NR^7$,

D représente un groupe imino, N-méthylimino, oxy, méthylène ou une liaison directe,

E représente un groupe carbonyle, $C=NR^7$, $C=N-OR^7$ ou sulfinyle, ou bien, lorsque F est une liaison, il peut également représenter un groupe hydroxyméthylène,

$R^7$ est défini comme $R^5$ à propos de $d_1$),
F représente un groupe oxy, imino, N-méthylimino ou une liaison directe,
$R^4$ est un groupe alkyle en $C_{1-6}$, cycloalkyle en $C_{3-6}$, aryle en $C_{6-12}$, aryl($C_{6-12}$)-alkyle($C_{1-5}$), aryloxy($C_{6-12}$)alkyle($C_{1-5}$), ou hétéroaryl($C_{3-9}$)alkyle($C_{1-5}$), le groupe alkyle pouvant chaque fois être substitué par 1 ou 2 restes identiques ou différents choisis parmi carboxy, amino, alkyl($C_{1-4}$)-amino, hydroxy, alcoxy en $C_{1-4}$, halogène, dialkyl($C_{1-4}$)amino, carbamoyle, sulfamoyle, alcoxy-($C_{1-4}$)carbonyle, aryle en $C_{6-12}$ et
aryl($C_{6-12}$)alkyle($C_{1-5}$), et les groupes aryle en $C_{6-12}$ et hétéroaryle en $C_{3-9}$ pouvant être substitués chacun par 1, 2 ou 3 restes identiques ou différents choisis parmi carboxy, cyano, amino, nitro, alkyl($C_{1-4}$)amino, hydroxy, alcoxy en $C_{1-4}$, halogène, dialkyl($C_{1-4}$)amino, carbamoyle, sulfamoyle et alcoxy($C_{1-4}$)carbonyle, et
$R^8$ est défini comme $R^3$ à propos de $c_1$),

ainsi que leurs sels physiologiquement compatibles, caractérisé en ce qu'on édifie le composé de manière connue à partir des fragments, on scinde éventuellement un ou plusieurs groupe(s) protecteur(s) temporairement introduit(s), on transforme éventuellement les fonctions carbonyle en analogues thia et on transforme éventuellement le composé de formule I ainsi obtenu en son sel physiologiquement compatible.

**2.** Procédé selon la revendication 1, caractérisé en ce qu'on prépare un composé de formule I dans lequel A représente un groupe alkyle en $C_{1-8}$ éventuellement substitué, alcanoyle en $C_{1-8}$ ou alcoxy-($C_{1-8}$)carbonyle éventuellement substitué, ou bien est défini comme dans la revendication 1 à propos de $a_3$).

**3.** Procédé selon une ou plusieurs des revendications 1 et 2, caractérisé en ce qu'on prépare un composé de formule I dans lequel $R^8$ est un atome d'hydrogène.

**4.** Procédé selon une ou plusieurs des revendications 1 à 3, caractérisé en ce qu'on prépare un composé de formule I dans lequel B représente un groupe carbonyle, thiocarbonyle ou $CR^5R^6$, où $R^5$ et $R^6$ sont H.

**5.** Procédé selon une ou plusieurs des revendications 1 à 4, caractérisé en ce qu'on prépare un composé de formule I dans lequel D représente un groupe imino, oxy ou méthylène.

**6.** Procédé selon une ou plusieurs des revendications 1 à 5, caractérisé en ce qu'on prépare un composé de formule I dans lequel E est un groupe carbonyle.

**7.** Procédé selon une ou plusieurs des revendications 1 à 6, caractérisé en ce qu'on prépare un composé de formule I dans lequel F est oxy ou une liaison directe.

**8.** Procédé selon une ou plusieurs des revendications 1 à 7, caractérisé en ce qu'on prépare un composé de formule I dans lequel $R^4$ représente un groupe aryle en $C_{6-12}$ éventuellement substitué ou un groupe aryl($C_{6-12}$)-alkyle($C_{1-5}$) éventuellement substitué dans la partie alkyle et/ou éventuellement substitué dans la partie aryle.

**9.** Procédé de préparation d'une composition pharmaceutique, contenant un composé de formule I selon une des revendications 1 à 8, caractérisé en ce qu'on met ce dernier sous une forme d'administration appropriée, en présence d'un véhicule physiologiquement acceptable et éventuellement d'autres auxiliaires ou additifs.